# EUROPEAN PATENT APPLICATION

(11) **EP 1 712 563 A1**
(43) Date of publication of application: **18.10.2006**
(21) Application number: 05709324.7
(22) Date of filing: 25.01.2005
(51) Int. Cl.: C07K 16/18, G01N 33/53, C12N 5/06, A61K 35/14, A61K 35/28, A61K 35/44, A61P 31/00, A61P 35/00, A61P 37/04

(54) **METHOD OF ISOLATING MONOCYTES**

(30) Priority: 27.01.2004 JP 2004018747
(71) Applicant: Medical and Biological Laboratories Co., Ltd., Nagoya-shi, Aichi 460-0002 (JP)
(72) Inventor: NAKAGAWA, Tomoko, Kamiina-gun, Nagano 399-4511 (JP); KUREI, Syunsuke, Ina-shi, Nagano 3960011 (JP); TOJI, Shingo, Ina-shi, Nagano 3960621 (JP); OKABE, Ayako, Kamiina-gun, Nagano 3960215 (JP); KUHARA, Motoki, Ina-shi, Nagano 396-0011 (JP); KISHI, Yoshiro, Ina-shi, Nagano 399-4501 (JP); YAHARA, Ichiro, Yokohoma-shi, Kanagawa 222-0012 (JP)
(74) Representative: Denison, Christopher Marcus
(86) International application number: PCT/JP2005/000923
(87) International publication number: WO 2005/070964

(57) **Abstract**

The present invention provides HIDE1 as novel monocyte markers. Since HIDE1 are membrane proteins, monocytes can be specifically detected by using antibodies that bind to HIDE1. Further, HIDE1-positive monocytes can also be collected from peripheral blood or the like using a cell sorter, magnet, or such. Monocytes that can be prepared based on the present invention are useful in cell immunotherapy.

SEQUENCE LISTING

<110>  MEDICAL AND BIOLOGICAL LABORATORIES CO., LTD.

<120>  Method of isolating monocytes

<130>  M3-A0307P

<150>  JP 2004-018747
<151>  2004-01-27

<160>  6

<170>  PatentIn version 3.1

<210>  1
<211>  669
<212>  DNA
<213>  Mus musculus

<220>
<221>  CDS
<222>  (1)..(669)
<223>

<400>  1
atg ccc tgg acc atc ctg ctg ttt gca tct ggc tcc ttg gcc atc cct        48
Met Pro Trp Thr Ile Leu Leu Phe Ala Ser Gly Ser Leu Ala Ile Pro
1               5                   10                  15

gca cca tcc atc tcc ttg gtg ccc ccc tac cca agc agc cac gag gac        96
Ala Pro Ser Ile Ser Leu Val Pro Pro Tyr Pro Ser Ser His Glu Asp
            20                  25                  30

ccc atc tac atc tcg tgc aca gcc cca ggg gac atc cta ggg gcc aat       144
Pro Ile Tyr Ile Ser Cys Thr Ala Pro Gly Asp Ile Leu Gly Ala Asn
        35                  40                  45

ttt acc ctg ttc cga ggg gga gag gtg gtc cag cta cta cag gcc ccc 192
Phe Thr Leu Phe Arg Gly Gly Glu Val Val Gln Leu Leu Gln Ala Pro
    50                  55                  60

tca gat cgg cct gat gta aca ttc aat gtg act ggt ggt ggc agt ggt 240
Ser Asp Arg Pro Asp Val Thr Phe Asn Val Thr Gly Gly Gly Ser Gly
65                  70                  75                  80

ggt ggc ggt gag gct gct ggg ggg aac ttc tgc tgt caa tat ggt gtg 288
Gly Gly Gly Glu Ala Ala Gly Gly Asn Phe Cys Cys Gln Tyr Gly Val
                85                  90                  95

atg ggt gag cac agt cag ccc cag ctg tcg gac ttc agc cag cag gtg 336
Met Gly Glu His Ser Gln Pro Gln Leu Ser Asp Phe Ser Gln Gln Val
            100                 105                 110

cag gtc tcc ttc cca gtc ccc acc tgg atc ttg gca ctc tcc ctg agc 384
Gln Val Ser Phe Pro Val Pro Thr Trp Ile Leu Ala Leu Ser Leu Ser
        115                 120                 125

ctg gct gga gct gtg ctg ttc tca ggg ctg gtg gcc atc aca gtg ctg 432
Leu Ala Gly Ala Val Leu Phe Ser Gly Leu Val Ala Ile Thr Val Leu
    130                 135                 140

gtg aga aaa gct aaa gcc aaa aac tta cag aag cag aga gag cgt gaa 480
Val Arg Lys Ala Lys Ala Lys Asn Leu Gln Lys Gln Arg Glu Arg Glu
145                 150                 155                 160

tcc tgc tgg gct cag atc aac ttc acc aat aca gac atg tcc ttt gat 528
Ser Cys Trp Ala Gln Ile Asn Phe Thr Asn Thr Asp Met Ser Phe Asp
                165                 170                 175

aac tct ctg ttt gct atc tcc acg aaa atg act cag gaa gac tca gtg 576
Asn Ser Leu Phe Ala Ile Ser Thr Lys Met Thr Gln Glu Asp Ser Val
            180                 185                 190

gca acc cta gac tca ggg cct cgg aag agg ccc acc tct gca tca tcc 624
Ala Thr Leu Asp Ser Gly Pro Arg Lys Arg Pro Thr Ser Ala Ser Ser
        195                 200                 205

tct ccg gag ccc cct gag ttc agc act ttc cgg gcc tgc cag tga 669
Ser Pro Glu Pro Pro Glu Phe Ser Thr Phe Arg Ala Cys Gln
    210                 215                 220

<210> 2
<211> 222
<212> PRT
<213> Mus musculus

<400> 2
Met Pro Trp Thr Ile Leu Leu Phe Ala Ser Gly Ser Leu Ala Ile Pro
1               5                   10                  15

Ala Pro Ser Ile Ser Leu Val Pro Pro Tyr Pro Ser Ser His Glu Asp
            20                  25                  30

Pro Ile Tyr Ile Ser Cys Thr Ala Pro Gly Asp Ile Leu Gly Ala Asn
        35                  40                  45

Phe Thr Leu Phe Arg Gly Gly Glu Val Val Gln Leu Leu Gln Ala Pro
    50                  55                  60

Ser Asp Arg Pro Asp Val Thr Phe Asn Val Thr Gly Gly Gly Ser Gly
65                  70                  75                  80

Gly Gly Gly Glu Ala Ala Gly Gly Asn Phe Cys Cys Gln Tyr Gly Val
                85                  90                  95

Met Gly Glu His Ser Gln Pro Gln Leu Ser Asp Phe Ser Gln Gln Val
            100                 105                 110

Gln Val Ser Phe Pro Val Pro Thr Trp Ile Leu Ala Leu Ser Leu Ser

115                 120                 125

Leu Ala Gly Ala Val Leu Phe Ser Gly Leu Val Ala Ile Thr Val Leu
    130                 135                 140

Val Arg Lys Ala Lys Ala Lys Asn Leu Gln Lys Gln Arg Glu Arg Glu
145                 150                 155                 160

Ser Cys Trp Ala Gln Ile Asn Phe Thr Asn Thr Asp Met Ser Phe Asp
                165                 170                 175

Asn Ser Leu Phe Ala Ile Ser Thr Lys Met Thr Gln Glu Asp Ser Val
            180                 185                 190

Ala Thr Leu Asp Ser Gly Pro Arg Lys Arg Pro Thr Ser Ala Ser Ser
        195                 200                 205

Ser Pro Glu Pro Pro Glu Phe Ser Thr Phe Arg Ala Cys Gln
    210                 215                 220

<210>  3
<211>  579
<212>  DNA
<213>  Mus musculus

<220>
<221>  CDS
<222>  (1)..(579)
<223>

<400>  3
atg ccc tgg acc atc ctg ctg ttt gca tct ggc tcc ttg gcc atc cct      48
Met Pro Trp Thr Ile Leu Leu Phe Ala Ser Gly Ser Leu Ala Ile Pro
1               5                   10                  15

gca cca tcc atc tcc ttg gtg ccc ccc tac cca agc agc cac gag gac      96

Ala Pro Ser Ile Ser Leu Val Pro Pro Tyr Pro Ser Ser His Glu Asp
            20                  25                  30

ccc atc tac atc tcg tgc aca gcc cca ggg gac atc cta ggg gcc aat 144
Pro Ile Tyr Ile Ser Cys Thr Ala Pro Gly Asp Ile Leu Gly Ala Asn
        35                  40                  45

ttt acc ctg ttc cga ggg gga gag gtg gtc cag cta cta cag gcc ccc 192
Phe Thr Leu Phe Arg Gly Gly Glu Val Val Gln Leu Leu Gln Ala Pro
    50                  55                  60

tca gat cgg cct gat gta aca ttc aat gtg act ggt ggt ggc agt ggt 240
Ser Asp Arg Pro Asp Val Thr Phe Asn Val Thr Gly Gly Gly Ser Gly
65                  70                  75                  80

ggt ggc ggt gag gct gct ggg ggg aac ttc tgc tgt caa tat ggt gtg 288
Gly Gly Gly Glu Ala Ala Gly Gly Asn Phe Cys Cys Gln Tyr Gly Val
                85                  90                  95

atg ggt gag cac agt cag ccc cag ctg tcg gac ttc agc cag cag gtg 336
Met Gly Glu His Ser Gln Pro Gln Leu Ser Asp Phe Ser Gln Gln Val
            100                 105                 110

cag gtc tcc ttc cca gct aaa gcc aaa aac tta cag aag cag aga gag 384
Gln Val Ser Phe Pro Ala Lys Ala Lys Asn Leu Gln Lys Gln Arg Glu
        115                 120                 125

cgt gaa tcc tgc tgg gct cag atc aac ttc acc aat aca gac atg tcc 432
Arg Glu Ser Cys Trp Ala Gln Ile Asn Phe Thr Asn Thr Asp Met Ser
    130                 135                 140

ttt gat aac tct ctg ttt gct atc tcc acg aaa atg act cag gaa gac 480
Phe Asp Asn Ser Leu Phe Ala Ile Ser Thr Lys Met Thr Gln Glu Asp
145                 150                 155                 160

tca gtg gca acc cta gac tca ggg cct cgg aag agg ccc acc tct gca 528
Ser Val Ala Thr Leu Asp Ser Gly Pro Arg Lys Arg Pro Thr Ser Ala

                165                 170                 175

tca tcc tct ccg gag ccc cct gag ttc agc act ttc cgg gcc tgc cag 576
Ser Ser Ser Pro Glu Pro Pro Glu Phe Ser Thr Phe Arg Ala Cys Gln
            180                 185                 190

tga 579

<210> 4
<211> 192
<212> PRT
<213> Mus musculus

<400> 4
Met Pro Trp Thr Ile Leu Leu Phe Ala Ser Gly Ser Leu Ala Ile Pro
1               5                   10                  15

Ala Pro Ser Ile Ser Leu Val Pro Pro Tyr Pro Ser Ser His Glu Asp
            20                  25                  30

Pro Ile Tyr Ile Ser Cys Thr Ala Pro Gly Asp Ile Leu Gly Ala Asn
        35                  40                  45

Phe Thr Leu Phe Arg Gly Gly Glu Val Val Gln Leu Leu Gln Ala Pro
    50                  55                  60

Ser Asp Arg Pro Asp Val Thr Phe Asn Val Thr Gly Gly Gly Ser Gly
65                  70                  75                  80

Gly Gly Gly Glu Ala Ala Gly Gly Asn Phe Cys Cys Gln Tyr Gly Val
                85                  90                  95

Met Gly Glu His Ser Gln Pro Gln Leu Ser Asp Phe Ser Gln Gln Val
            100                 105                 110

Gln Val Ser Phe Pro Ala Lys Ala Lys Asn Leu Gln Lys Gln Arg Glu

115                 120                 125

Arg Glu Ser Cys Trp Ala Gln Ile Asn Phe Thr Asn Thr Asp Met Ser
    130                 135                 140

Phe Asp Asn Ser Leu Phe Ala Ile Ser Thr Lys Met Thr Gln Glu Asp
145                 150                 155                 160

Ser Val Ala Thr Leu Asp Ser Gly Pro Arg Lys Arg Pro Thr Ser Ala
                165                 170                 175

Ser Ser Ser Pro Glu Pro Pro Glu Phe Ser Thr Phe Arg Ala Cys Gln
            180                 185                 190

<210>  5
<211>  690
<212>  DNA
<213>  Homo sapiens

<220>
<221>  CDS
<222>  (1)..(690)
<223>

<400>  5
atg ccc tgg acc atc ttg ctc ttt gca gct ggc tcc ttg gcg atc cca      48
Met Pro Trp Thr Ile Leu Leu Phe Ala Ala Gly Ser Leu Ala Ile Pro
1               5                   10                  15

gca cca tcc atc cgg ctg gtg ccc ccg tac cca agc agc caa gag gac      96
Ala Pro Ser Ile Arg Leu Val Pro Pro Tyr Pro Ser Ser Gln Glu Asp
            20                  25                  30

ccc atc cac atc gca tgc atg gcc cct ggg aac ttc ccg ggg gcg aat      144
Pro Ile His Ile Ala Cys Met Ala Pro Gly Asn Phe Pro Gly Ala Asn
        35                  40                  45

ttc aca ctg tat cga ggg ggg cag gtg gtc cag ctc ctg cag gcc ccc 192
Phe Thr Leu Tyr Arg Gly Gly Gln Val Val Gln Leu Leu Gln Ala Pro
    50                  55                  60

acg gac cag cgc ggg gtg aca ttt aac ctg agc ggc ggc agc agc aag 240
Thr Asp Gln Arg Gly Val Thr Phe Asn Leu Ser Gly Gly Ser Ser Lys
65                  70                  75                  80

gct cca ggg gga ccc ttc cac tgc cag tat gga gtg tta ggt gag ctc 288
Ala Pro Gly Gly Pro Phe His Cys Gln Tyr Gly Val Leu Gly Glu Leu
                85                  90                  95

aac cag tcc cag ctg tca gac ctc agc gag ccc gtg aac gtc tcc ttc 336
Asn Gln Ser Gln Leu Ser Asp Leu Ser Glu Pro Val Asn Val Ser Phe
            100                 105                 110

cca gtg ccc act tgg atc ttg gtg ctc tcc ctg agc ctg gct ggt gcc 384
Pro Val Pro Thr Trp Ile Leu Val Leu Ser Leu Ser Leu Ala Gly Ala
        115                 120                 125

ctc ttc ctc ctt gct ggg ctg gtg gct gtt gcc ctg gtg gtc aga aaa 432
Leu Phe Leu Leu Ala Gly Leu Val Ala Val Ala Leu Val Val Arg Lys
    130                 135                 140

gtt aaa ctc aga aat tta cag aag aaa aga gat cga gaa tcc tgc tgg 480
Val Lys Leu Arg Asn Leu Gln Lys Lys Arg Asp Arg Glu Ser Cys Trp
145                 150                 155                 160

gcc cag att aac ttc gac agc aca gac atg tcc ttc gat aac tcc ctg 528
Ala Gln Ile Asn Phe Asp Ser Thr Asp Met Ser Phe Asp Asn Ser Leu
                165                 170                 175

ttt acc gtc tcc gcg aaa acg atg cca gaa gaa gac ccg gcc acc ttg 576
Phe Thr Val Ser Ala Lys Thr Met Pro Glu Glu Asp Pro Ala Thr Leu
            180                 185                 190

gat gat cac tca ggc acc act gcc acc ccc agc aac tcc agg acc cgg          624
Asp Asp His Ser Gly Thr Thr Ala Thr Pro Ser Asn Ser Arg Thr Arg
        195                 200                 205

aag agg ccc act tcc acg tcc tcc tcg cct gag acc ccc gaa ttc agc          672
Lys Arg Pro Thr Ser Thr Ser Ser Ser Pro Glu Thr Pro Glu Phe Ser
    210                 215                 220

act ttc cgg gcc tgc cag                                                  690
Thr Phe Arg Ala Cys Gln
225                 230

<210>  6
<211>  230
<212>  PRT
<213>  Homo sapiens

<400>  6
Met Pro Trp Thr Ile Leu Leu Phe Ala Ala Gly Ser Leu Ala Ile Pro
1               5                   10                  15

Ala Pro Ser Ile Arg Leu Val Pro Pro Tyr Pro Ser Ser Gln Glu Asp
            20                  25                  30

Pro Ile His Ile Ala Cys Met Ala Pro Gly Asn Phe Pro Gly Ala Asn
        35                  40                  45

Phe Thr Leu Tyr Arg Gly Gly Gln Val Val Gln Leu Leu Gln Ala Pro
    50                  55                  60

Thr Asp Gln Arg Gly Val Thr Phe Asn Leu Ser Gly Gly Ser Ser Lys
65                  70                  75                  80

Ala Pro Gly Gly Pro Phe His Cys Gln Tyr Gly Val Leu Gly Glu Leu
                85                  90                  95

Asn Gln Ser Gln Leu Ser Asp Leu Ser Glu Pro Val Asn Val Ser Phe
            100                 105                 110

Pro Val Pro Thr Trp Ile Leu Val Leu Ser Leu Ser Leu Ala Gly Ala
        115                 120                 125

Leu Phe Leu Leu Ala Gly Leu Val Ala Val Ala Leu Val Val Arg Lys
    130                 135                 140

Val Lys Leu Arg Asn Leu Gln Lys Lys Arg Asp Arg Glu Ser Cys Trp
145                 150                 155                 160

Ala Gln Ile Asn Phe Asp Ser Thr Asp Met Ser Phe Asp Asn Ser Leu
                165                 170                 175

Phe Thr Val Ser Ala Lys Thr Met Pro Glu Glu Asp Pro Ala Thr Leu
            180                 185                 190

Asp Asp His Ser Gly Thr Thr Ala Thr Pro Ser Asn Ser Arg Thr Arg
        195                 200                 205

Lys Arg Pro Thr Ser Thr Ser Ser Ser Pro Glu Thr Pro Glu Phe Ser
    210                 215                 220

Thr Phe Arg Ala Cys Gln
225                 230

## Description

### Technical Field

The present invention relates to antibodies for detecting monocyte markers, and uses thereof.

### Background Art

Monocytes are cells which migrate in the blood and which have phagocytotic activity, belonging to the group of mononuclear phagocytes. Once differentiated, monocytes remain in the bone marrow for only short time before entering the circulatory system, where they remain for several days. Monocytes then infiltrate tissues and body cavities, and differentiate into macrophages and dendritic cells. Monocytes are known to increase in the circulatory system in inflammatory diseases, post-transplantation rejection reactions, infectious disease recovery phases, monocytic leukemia, and such.

Immunotherapy is a therapeutic method aiming to potentiate the immunological function of patients using substances that activate immune cells, cytokines, antibodies, and the like. Immunotherapy is being noted as an auxiliary therapy to improve the therapeutic effect of other treatments or to prevent recurrences and the like. A representative immunotherapy includes cell immunotherapy. Typically, cell immunotherapy comprises the steps of collecting immune cells from a patient; growing and activating the *cells in vitro;* and then returning the cells to the patient. Such cells to be used in cell immunotherapy can include, for example, lymphocytes and dendritic cells.

One antitumor immunotherapy using lymphocytes is a known method which uses peripheral blood lymphocytes, tumor tissue-infiltrating lymphocytes, and the like, which are activated or grown using cytokines (lymphokine-activated killer (LAK) cell therapy; J. Immunol. (1984) 132: 2123-8; J. Exp. Med. (1982) 155: 1823-41). In LAK therapy, for example, lymphocytes are separated from a patient's blood; a cytokine such as interleukin-2 (IL-2) is added to those peripheral blood lymphocytes that have not received antigen stimulation, and lymphokine-activated killer cells (LAK cells) with strong antitumor activity are prepared; and then the LAK cells are returned to the patient.

From the 1990s great strides have been made in the study of dendritic cells (DCs). DCs are known as cells with strong antigen-presenting activity. Since DCs are the most efficient cells in terms of presenting antigens and activating T lymphocytes, their application to cell immunotherapy has been proposed. Methods of cell immunotherapy using dendritic cells are, for example, *ex vivo* ingestion of tumor cell antigens (cancer specific antigens) by a patient's dendritic cells to potentiate cancer antigen-presenting ability; and then the return of these cells to the patient to induce an immune response against the patient's cancer. When dendritic cells that have phagocytosed antigens *ex vivo* are administered to patients, they are thought to induce helper T *cells in vivo,* and these helper T cells activate killer T cells and natural killer cells, thereby potentiating the patient's ability to eliminate the cancer.

The first clinical application of this kind of cell immunotherapy using dendritic cells targeted melanomas. In immune responses that take place upon invasion of a foreign antigen into the body, dendritic cells that have phagocytosed a foreign antigen migrate to the lymph nodes and present the antigen to lymphocytes. Thus, therapeutic methods comprising the injection of dendritic cells directly into lesions such as tumors are also being used (dendritic cell injection (DCI)). Also in practical use are methods that comprise mixing dendritic cells and activated lymphocytes to activate both cells, and then administering these cells to patients (DCAT therapy).

Examples of other therapeutic methods using blood cells include macrophage-based therapies for spinal cord damage. There are reports that axon regrowth is induced by direct administration of macrophages to the lesion site of rats whose spinal cord has been cut. Methods for treating human paralysis caused by spinal cord damage, which comprise injecting activated autologous macrophages into spinal cord parenchymal tissues, are also being assessed from a clinical viewpoint.

Many secretory and membrane proteins are known to have receptor functions, and to transmit cellular signals that initiate various cellular responses upon ligand binding. In general, many ligands involved in such cellular responses are also pharmaceutically important. Thus, many studies are being performed to identify ligands and receptors. Patent Documents 1 and 2 also aim to identify this kind of receptor, and describe the preparation of cDNAs encoding the transmembrane protein called "human TANGO353" from among clones in a mixed lymphocyte reaction library. Based only on its origin, Patent Documents 1 and 2 state that TANGO353 can be used to proliferate, differentiate, and activate T or B cells, and to relieve symptoms involved in the functional abnormality of these cells, and so on. However, Patent Documents 1 and 2 do not make more detailed investigation of the protein's distribution of expression, actual function, and the like, and thus the relationship between TANGO353 and monocytes is unclear from the descriptions of these Documents.

At present, an enormous volume of genome sequence information has been disclosed. Gene expression profiling is thought to be greatly useful in elucidating biological events involving genes whose sequences have been determined. High coverage expression profiling (HiCEP) technology is a method developed based on amplified fragment length polymorphism (AFLP; Vos et al., Nucleic Acids Res. (1995) 23: 4407-17). Expression profiling using HiCEP technology does not require sequence information and can detect non-coding transcripts, and known and unidentified genes.
[Patent Document 1] U.S. Patent Application NO. 2002/0055139
[Patent Document 2] WO 01/09162
[Non-patent Document 1] Fukumura et al., Nucleic Acids Res. (2003) 31: e94

### Disclosure of the Invention

Dendritic cells and macrophages to be used in cell immunotherapy or such can be induced to differentiate from monocytes. Thus, methods using autologous peripheral blood monocytes separated from patients are in wide clinical use. In methods using peripheral blood monocytes, the monocytes are first separated by apheresis or density centrifugation. Such physical methods require repeated centrifugation, and are problematic in that they significantly damage cells and further increase the chance of bacterial contamination. Some surface antigens, including CD14, CD11b, and CD33, are known as monocyte markers; however, more specific monocyte markers are required. Thus, an objective of the present invention is to identify proteins to become markers that are highly expressed in monocytes.

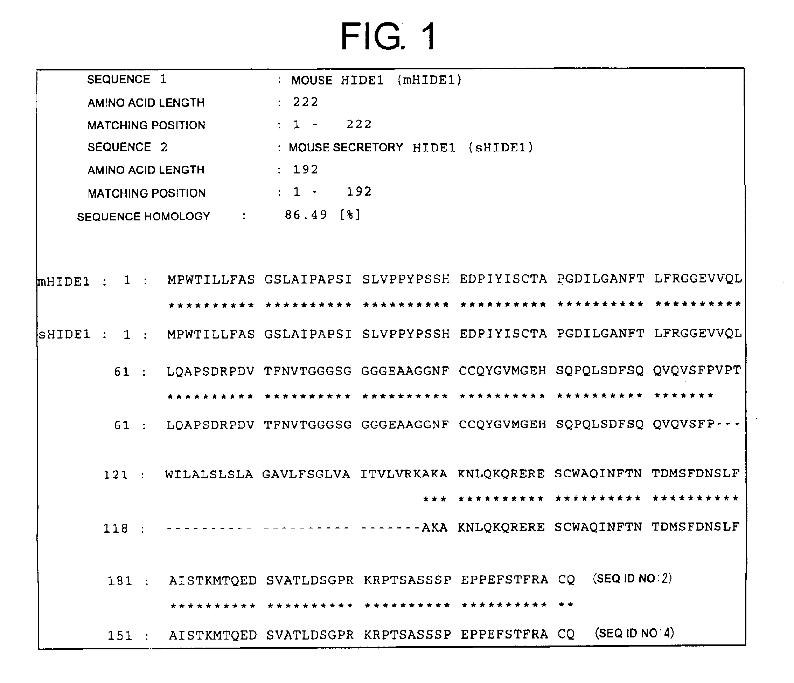

The mRNAs of about 60,000 and 57,000 genes were respectively collected from immature and mature dendritic cells of mouse spleens. Differences in expression levels between the respective genes were determined using HiCEP technology (Non-patent Document 1 ). As a result, "High expression Gene of Immature Dendritic cells 1 (HIDE1)", a single transmembrane protein gene expressed at high levels in immature dendritic cells, was identified (SEQ ID NO: 1; the amino acid sequence encoded by the gene is shown in SEQ ID NO: 2). Meanwhile, a splice variant that did not comprise a transmembrane domain was recovered during PCR cloning of the full-length gene. This suggests the existence of a secretory HIDE1 (soluble HIDEI: sHIDE1) (nucleotide sequence: SEQ ID NO: 3; amino acid sequence: SEQ ID NO: 4). The amino acid sequences of membrane HIDE1 and secretory HIDE1 are compared in Fig. 1.

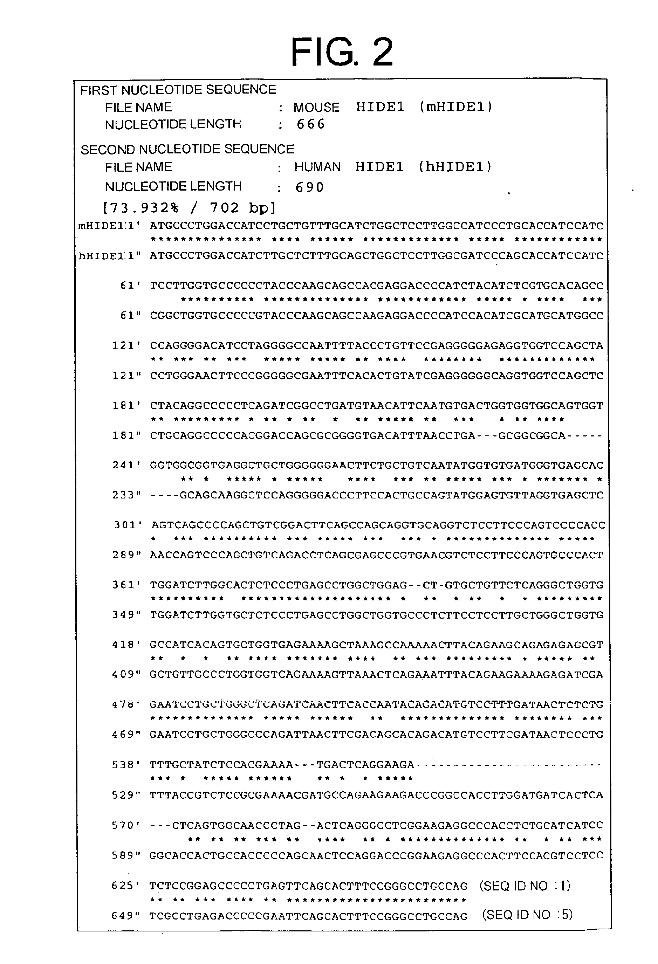

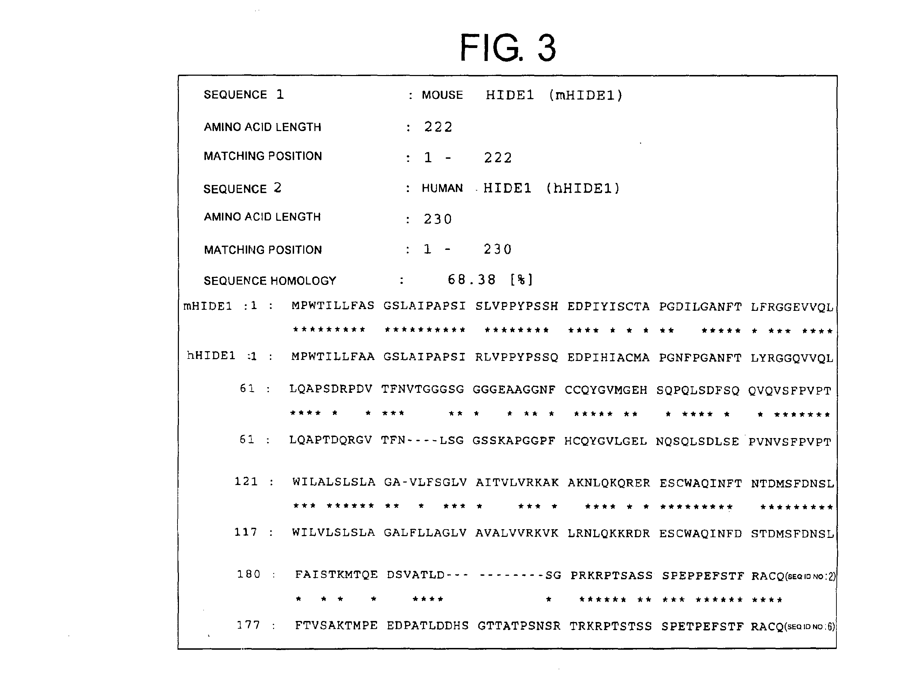

HIDE1 was expressed at high levels in murine immature dendritic cells, and other researchers had already disclosed a human homolog of HIDE1 in a database (XM_172995). The nucleotide sequence of human HIDE1 and the amino acid sequence encoded by the same are shown in SEQ ID NOs: 5 and 6, respectively (Figs. 2 and 3). The nucleotide sequence homology and amino acid sequence homology between mouse and human HIDE1 were found to be 73.9% and 68.4%, respectively.

The human HIDE1 shown by the present invention to be a novel monocyte marker has a sequence 100% identical to that of the TANGO353 described in Patent Documents 1 and 2. However, Patent Documents 1 and 2 describe the cloning of TANGO353 from a mixture comprising T and B lymphocytes, and only suggest the possibility that TANGO353 is involved in the differentiation and growth of T and B lymphocytes based on its origin. Specifically, these documents neither describe nor suggest the expression of TANGO353 in monocytes, nor its use as a monocyte marker.

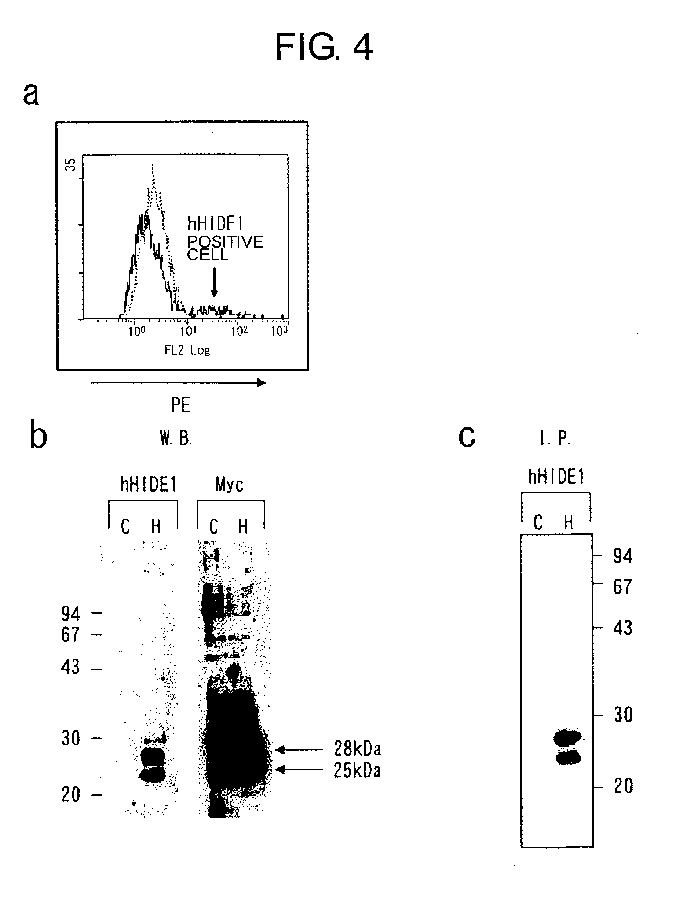

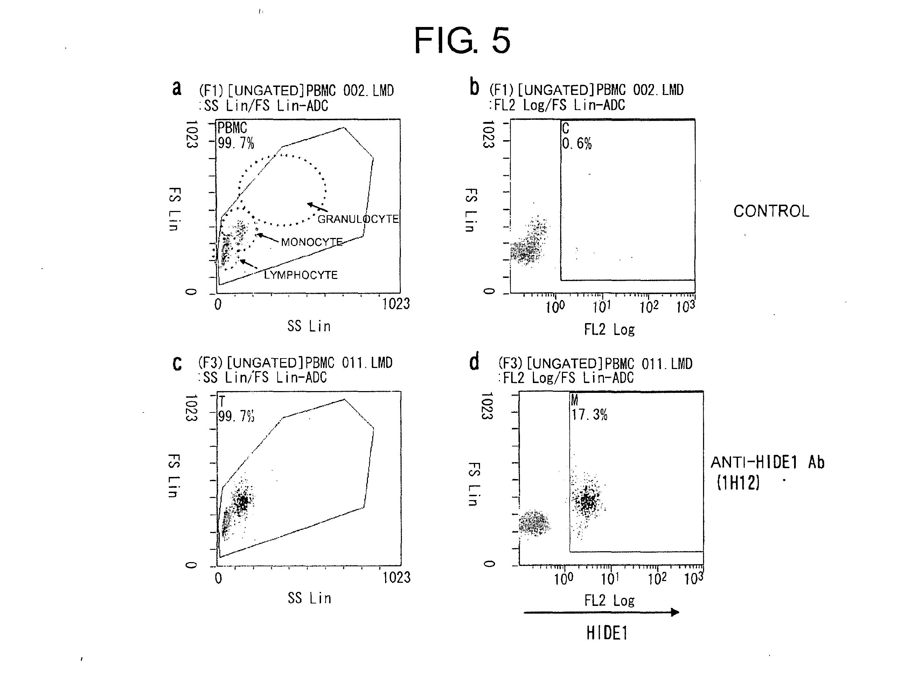

Human HIDE1 gene was cloned from a placental cDNA library, and that gene product was used to immunize mice, producing a monoclonal antibody (anti-HIDE1 antibody) that can be used in FCM, WB, and immunoprecipitation (Fig. 4). Then, human peripheral blood mononuclear cells (PBMCs) were stained with the anti-HIDE1 antibody, and the results showed that HIDE1 was expressed mainly in monocytes, but not in lymphocytes. Slight expression was also observed in granulocytes, but with much weaker staining than the monocytes (Fig. 5). Furthermore, double staining was carried out using the anti-HIDE1 antibody and another monocyte marker (CD14, CD11b, or CD33). The results showed that the anti-HIDE1 antibody stained monocytes more broadly than CD14, and monocytes could be stained more specifically when using HIDE 1 as a marker than when using CD 11b.

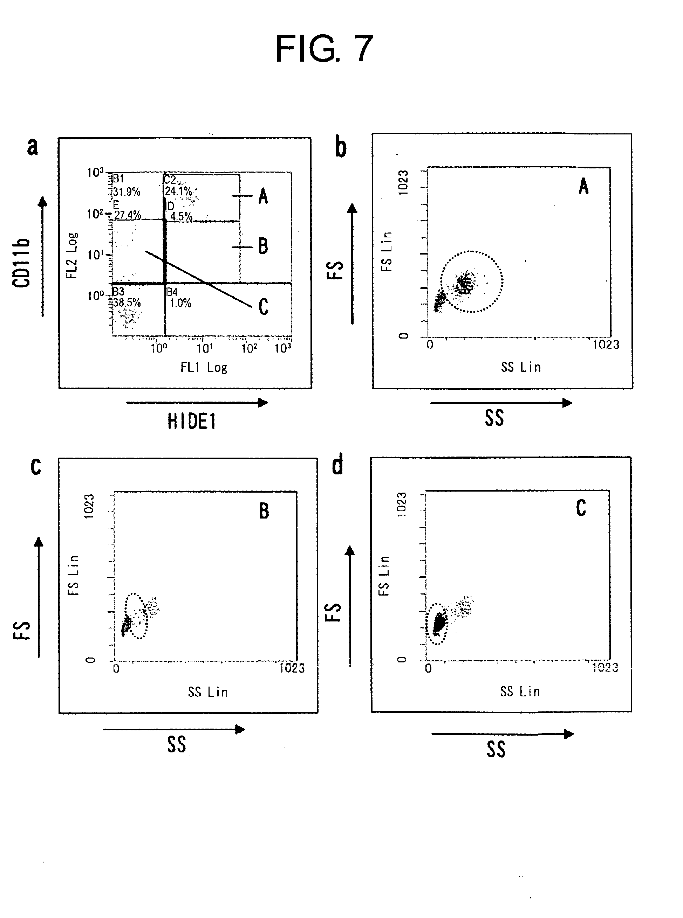

Furthermore, HIDE1 expression was found in human monocytic cultured cell lines such as HL60, U937, and THP-1. It was thus revealed that HIDE1 could be used as a marker for monocytes, including such cultured cell lines. When THP-1, a monocytic cultured cell line, was differentiated into macrophage-like cells using phorbol ester as a differentiation-inducing factor, HIDE1 expression markedly decreased (Fig. 7d). This result strongly suggests that HIDE1 is expressed specifically in monocytes, and emphasizes that HIDE1 can be used as a monocyte marker.

As described above, the present invention demonstrated that HIDE1 could be used as a specific monocyte marker. Monocytes developed and differentiated from bone marrow stem cells *in vivo* are known to migrate from blood into tissues, and then differentiate into dendritic cells (DCs) and macrophages with more activated functions. Thus, not only can monocytes be detected and isolated by screening cells using the HIDE1 monocyte markers of the present invention as an indicator, but DCs and macrophages can also be prepared by differentiating the isolated monocytes *in vitro* using known techniques. Further, since HIDE1 is not expressed in lymphocytes, HIDE1 can also be used as a tool to enrich lymphocytes by removing HIDE1-positive cells from peripheral blood using an anti-HIDE1 antibody. Thus, the present invention provides the following antibodies and uses thereof:
[1] an antibody for detecting a monocyte marker, which binds to a protein or a polypeptide selected from the group of:
   (1) an HIDE1 protein,
   (2) a protein encoded by a nucleotide sequence that hybridizes to a complementary sequence of an HIDE1 gene under stringent conditions, and
   (3) a polypeptide fragment with at least eight amino acid residues, wherein the fragment is derived from the protein of the above (1) or (2);
[2] a method for detecting a monocyte, which comprises the steps of:
   (1) contacting the antibody of [1] with a blood cell sample predicted to comprise a monocyte, and
   (2) detecting a blood cell that has bound to the antibody in step (1);
[3] a method for isolating a monocyte, which comprises the steps of:
   (1) contacting the antibody of [1] with a blood cell sample predicted to comprise a monocyte, and
   (2) collecting a blood cell that has bound to the antibody in step (1);
[4] the method of [2] or [3], wherein the blood cell sample is peripheral blood, cord blood, or bone marrow;
[5] a kit for detecting and/or isolating a monocyte, which comprises the antibody of [1];
[6] a method for inducing a dendritic cell from a monocyte *in vitro,* which comprises the steps of:
   (1) culturing a monocyte in the presence of a differentiation inducing factor for a dendritic cell, and
   (2) contacting a cell cultured in step (1) with the antibody of [1], detecting HIDE1 expression, and judging that the differentiation of a monocyte into a dendritic cell is induced when the expression level of HIDE 1 is reduced;
[7] the method of [6], wherein the differentiation inducing factor for a dendritic cell is a combination of GM-CSF and IL-4;
[8] a method for inducing a macrophage from a monocyte *in vitro,* which comprises the steps of:
   (1) culturing a monocyte in the presence of a differentiation inducing factor for a macrophage, and
   (2) contacting a cell cultured in step (1) with the antibody of [1], detecting HIDE 1 expression, and judging that the differentiation of a monocyte into a macrophage-like cell is induced when the expression level of HIDE1 is reduced;
[9] the method of [8], wherein the differentiation inducing factor for a macrophage is phorbol ester;
[10] a method for obtaining a dendritic cell, which comprises the steps of:
   (1) contacting a sample of collected blood cells with the antibody of [1],
   (2) collecting a blood cell that has bound to the antibody in step (1),
   (3) culturing the blood cell collected in step (2) in the presence of a differentiation inducing factor for a dendritic cell,
   (4) contacting the cell cultured in step (3) with the antibody of [1], detecting HIDE1 expression, and judging that a monocyte is differentiated into a dendritic cell when the expression level of HIDE1 is reduced, and
   (5) isolating as a dendritic cell the cell judged to be differentiated in step (4);
[11] the method of [10], which further comprises the step of allowing the isolated dendritic cell to ingest an antigen;
[12] the method of [10], wherein the isolated dendritic cell is used to prevent and/or treat a tumor.
[13] the method of [12], which further comprises the step of allowing the isolated dendritic cell to ingest a tumor-specific antigen;
[14] the method of [10], wherein the isolated dendritic cell is used to prevent and/or treat an autoimmune disease, or to relieve rejection after an organ transplantation;
[15] a method for obtaining a macrophage, which comprises the steps of:
   (1) contacting a sample of collected blood cells with the antibody of [1],
   (2) collecting a blood cell that has bound to the antibody in step(1),
   (3) culturing the blood cell collected in step (2) in the presence of a differentiation inducing factor for a macrophage,
   (4) contacting the cell cultured in step (3) with the antibody of [1], detecting HIDE1 expression, and judging that a monocyte is differentiated into a macrophage-like cell when the expression level of HIDE 1 is reduced, and
   (5) isolating as a macrophage the cell judged to be differentiated in step (4);
[16] the method of [15], which further comprises the step of activating the isolated cell;
[17] the method of [15] or [16], wherein the isolated macrophage is used to treat a spinal cord damage, and/or to treat and/or prevent a tumor, infectious disease, autoimmune disease, or immunodeficiency disease;
[18] a method for collecting a lymphocyte, which comprises the steps of:
   (1) contacting the antibody of [1] with a blood cell sample predicted to comprise a lymphocyte, and
   (2) collecting a blood cell that did not bind to the antibody in step (1);
[19] a method for obtaining an activated lymphocyte, which comprises the steps of:
   (1) contacting the antibody of [1] with a blood cell sample predicted to comprise a lymphocyte,
   (2) collecting as a lymphocyte a blood cell that is not bound to the antibody,
   (3) culturing the lymphocyte collected in step (2), and
   (4) activating the lymphocyte cultured in step (3) and collecting the activated lymphocyte;
[20] the method of [18] or [19], wherein the blood cell sample is peripheral blood, cord blood, or bone marrow; and
[21] the method of [19], wherein the activated lymphocyte is used to prevent and/or treat a tumor or infectious disease.

Monocytes, dendritic cells, macrophages, and lymphocytes can be prepared by using the antibodies of the present invention. Thus, the present invention also provides therapeutic and/or preventive methods using these cells. Specifically, the present invention relates to uses in the production of pharmaceutical compositions to treat and/or prevent tumors by contacting an antibody of the present invention with a patient blood cell sample such as peripheral blood, cord blood, or bone marrow; isolating monocytes bound to the antibody; and administering patients with the dendritic cells obtainable upon treating the monocytes with appropriate cytokines, after activation as required.

Alternatively, the present invention relates to uses of antibodies or fragments comprising a variable region thereof, which bind to proteins or polypeptides selected from (1) to (3), as antibodies for detecting monocyte markers. In addition, the present invention relates to uses of antibodies or fragments comprising a variable region thereof, which bind to proteins or polypeptides selected from (1) to (3), in the production of kits for detecting and/or isolating monocytes.
(1) an HIDE 1 protein;
(2) a protein encoded by a nucleotide sequence that hybridizes under stringent conditions to a complementary sequence of an HIDE1 gene; and
(3) a polypeptide fragment having at least eight amino acid residues, which is a fragment of a protein of the above (1) or (2).

In addition, the present invention provides methods for preventing and/or treating tumors, which comprise the step of administering those dendritic cells isolated by using an antibody described above. Alternatively, the present invention relates to uses of the dendritic cells isolated using an antibody described above in the production of pharmaceutical compositions for preventing and/or treating tumors.

Furthermore, the present invention provides methods for preventing and/or treating autoimmune diseases, or relieving rejection after organ transplantation, which comprise the step of administering the dendritic cells isolated using an antibody described above. Alternatively, the present invention relates to uses of the dendritic cells, isolated using an antibody described above, in the production of pharmaceutical compositions for preventing and/or treating autoimmune diseases, or for relieving rejection after organ transplantation.

Moreover, the present invention provides methods for treatment of spinal cord damage and treatment and/or prevention of tumors, infectious diseases, autoimmune diseases, and immunodeficiency diseases, which comprise the step of administering the macrophages isolated using an antibody described above. Alternatively, the present invention relates to uses of the macrophages isolated using an antibody described above in the production of pharmaceutical compositions for treating spinal cord damage and treating and/or preventing tumors, infectious diseases, autoimmune diseases, and immunodeficiency diseases.

In addition, the present invention provides methods for preventing and/or treating tumors or infectious diseases, which comprise the step of administering the activated lymphocytes isolated using an antibody described above. Alternatively, the present invention relates to uses of the activated lymphocytes isolated using an antibody described above in the production of pharmaceutical compositions for preventing and/or treating tumors or infectious diseases.

### Brief Description of the Drawings

Fig. 1 is a diagram comparing the amino acid sequences of mouse HIDE1 (mHIDE1; SEQ ID NO: 2) and mouse secretory HIDE1 (sHIDE1; SEQ ID NO: 4). Sequence homology was found to be 86.5%. sHIDE1 lacks the transmembrane domain comprised in mHIDE1.
Fig. 2 is a diagram comparing the mRNA sequences of mouse HIDE1 (mHIDE1; SEQ ID NO: 1) and human HIDE1 (hHIDE1; SEQ ID NO: 5). Sequence homology was found to be 73.9%.
Fig. 3 is a diagram comparing the amino acid sequences of mouse HIDE1 (mHIDE1; SEQ ID NO: 2) and human HIDE1 (hHIDE1; SEQ ID NO: 6). Sequence homology was found to be 68.4%.
Fig. 4 shows the results of confirming acquisition of anti-HIDE1 antibody. (a) shows the results of flow cytometry (FCM) using 293T transiently expressing hHIDE1 and wild type 293T. The solid line indicates the reaction of an anti-HIDE1 antibody (1H12) with 293T transiently expressing hHIDE1; the broken line indicates the reaction with wild type 293T. (b) shows the results of Western blotting of 293T(H) expressing hHIDE1 and 293T(C) expressing another Myc-tag fusion protein using an anti-HIDE1 antibody (3F12). An anti-Myc-tag antibody (MBL) was used as the positive control. Arrows indicate the position of the positive control bands. (c) shows the results of immunoprecipitation for 293T(H) expressing hHIDE1 and 293T(C) expressing another protein using an anti-HIDE1 antibody (3H3).
Fig. 5 shows the results of (ungated) flow cytometry (FCM) analysis of a peripheral blood mononuclear cell (PBMC) fraction using an anti-HIDE1 antibody (1H12). (a) shows the results of two-dimensional fractionation of PBMCs in terms of cell size (FS) and light scattering (SS). From the left, cell populations were categorized into lymphocytes, monocytes, and granulocytes. (b) shows the results of staining PBMCs with an isotopic control. (c) shows the results of two-dimensional fractionation of PBMCs with FS and SS. Area M in plot (d) indicates the anti-HIDE1 antibody-positive cells. (d) shows the results of FCM analysis of PBMCs stained with the anti-HIDE1 antibody. HIDE1-positive cells were gated, and are shown as "area M".
Fig. 6 shows the results of double staining with an anti-HIDE1 antibody and an antibody against CD 14, a monocyte marker. Each plot shows the following analysis results:
   (a) Each cell population separated by double staining was gated (A, B, and C).
   (b) The whole PBMC was fractionated with FS and SS (gray). Cell population A from plot (a) (CD14(++) & anti-HIDE1 antibody (+)) is shown in black (enclosed by the broken line).
   (c) The whole PBMC was fractionated with FS and SS (gray). Cell population B from plot (a) (CD14(+) & anti-HIDE1 antibody (+)) is shown in black (enclosed by the broken line).
   (d) The whole PBMC was fractionated with FS and SS (gray). Cell population C from plot (a) (CD14(-) & anti-HIDE1 antibody (+)) is shown in black (enclosed by the broken line).
Fig. 7 shows the results of double staining with an anti-HIDE1 antibody and an antibody against CD11b, a monocyte marker. Each plot shows the following analysis results:
   (a) Each cell population separated by double staining was gated (A, B, and C).
   (b) The whole PBMC was fractionated with FS and SS (gray). Cell population A from plot (a) (CD11b(++) & anti-HIDE1 antibody (+)) is shown in black (enclosed by the broken line).
   (c) The whole PBMC was fractionated with FS and SS (gray). Cell population B from plot (a) (CD1 1b(+) & anti-HIDE 1 antibody (+)) is shown in black (enclosed by the broken line).
   (d) The whole PBMC was fractionated with FS and SS (gray). Cell population C from plot (a) (CD11b(+) & anti-HIDE1 antibody (-)) is shown in black (enclosed by the broken line).

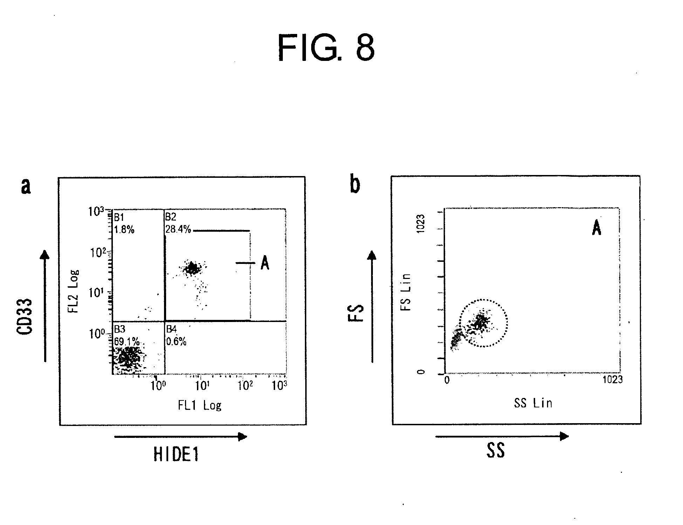

Fig. 8 shows the results of double staining with an anti-HIDE1 antibody and an antibody against CD33, a monocyte marker. Each plot shows the following analysis results:
   (a) The double positive cell population was gated (gate A).
   (b) The whole PBMC was fractionated with FS and SS (gray). Cell population A from plot (a) (CD33(+) & anti-HIDE1 antibody (+)) is shown in black (enclosed by the broken line).

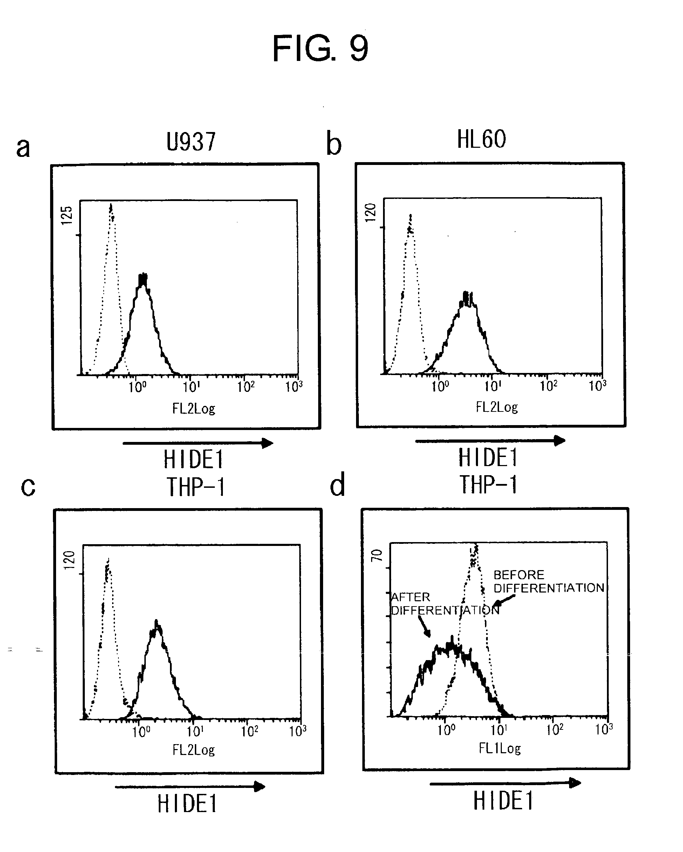

Fig. 9 shows the results of staining cells from human monocytic cultured lines using an anti-HIDE1 antibody (1H12). (a) used U937, (b) used HL60, and (c) and (d) used THP-1 cultured cells. In (a) to (c), the solid line shows the reaction using anti-HIDE1 antibody and the broken line shows the reaction using the isotopic control. In (d), the broken line indicates the reaction between the anti-HIDE1 antibody and the cells before differentiation, and the solid line indicates the reaction between the antibody and the differentiated cells.

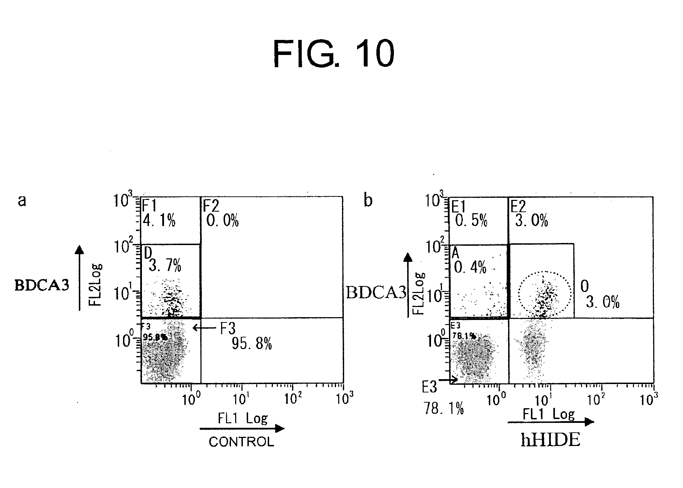

Fig. 10 shows the results of double staining human peripheral blood mononuclear cells (PBMCs) with an anti-hHIDE 1 antibody (1H12) and a marker for bone marrow-derived dendritic cell line (BDCA3):
   (a) double staining of PBMCs with the anti-HIDE1 antibody (1H12) and an isotype control
   (b) double staining of PBMCs with the anti-HIDE1 antibody (1H12) and BDCA3.

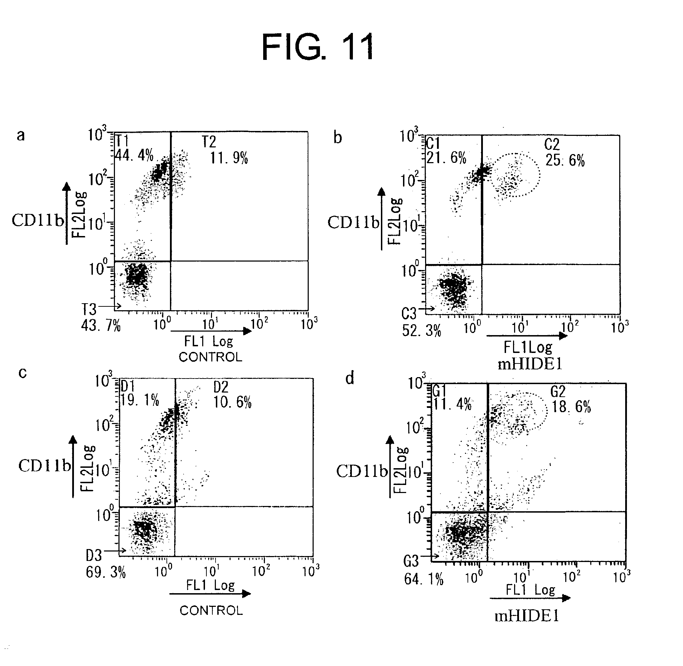

Fig. 11 shows the results of double staining mouse peripheral blood mononuclear cells (PBMCs) and mouse spleen cells with an anti-mHIDE1 antibody (5F8) and a monocyte marker (CD11b):
   (a) double staining of mouse peripheral blood mononuclear cells (PBMCs) with an anti-HIDE1 antibody (1H12) and an isotype control
   (b) double staining of mouse peripheral blood mononuclear cells (PBMCs) with an anti-HIDE1 antibody (1H12) and a monocyte marker (CD11b)
   (c) double staining of mouse spleen cells with an anti-HIDE1 antibody (1H12) and an isotype control
   (d) double staining of mouse spleen cells with an anti-HIDE1 antibody (1H12) and a monocyte marker (CD 11b).

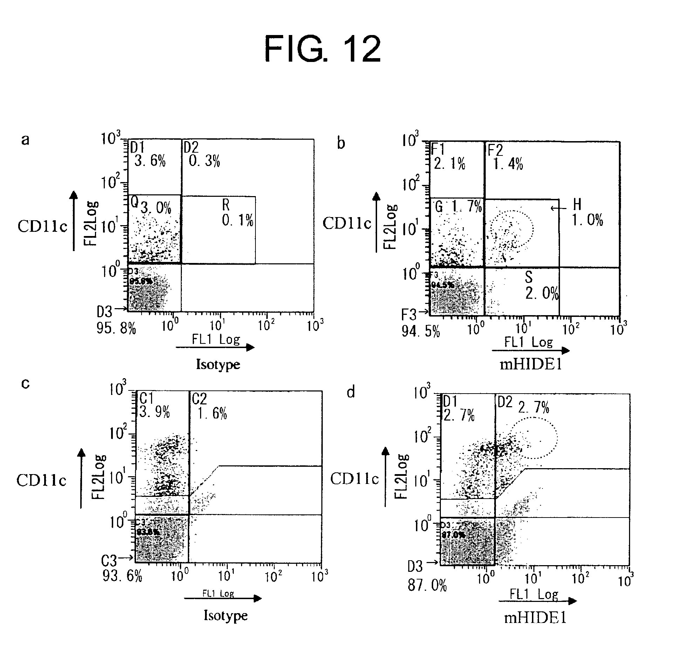

Fig. 12 shows the results of double staining mouse peripheral blood mononuclear cells (PBMCs) and mouse spleen cells using an anti-mHIDE1 antibody (5F8) and a dendritic cell marker (CD11c):
   (a) double staining of mouse PBMCs with an anti-HIDE1 antibody (1H12) and an isotype control
   (b) double staining of mouse PBMCs with an anti-HIDE1 antibody (1H12) and a dendritic cell marker (CD11c)
   (c) double staining of mouse spleen cells with an anti-HIDE1 antibody (1H12) and an isotype control
   (d) double staining of mouse spleen cells with an anti-HIDE1 antibody (1H12) and a dendritic cell marker (CD 11c).

### Best Mode for Carrying Out the Invention

### [Antibodies for detecting monocyte markers]

The present invention revealed that monocytes could be more broadly stained using HIDE1 as a marker, than CD14, a known monocyte marker, and also revealed that more specific staining of monocytes could be achieved with HIDE1 than with CD11b. Thus, the present invention provides antibodies for detecting monocyte markers (HIDE1). Antibodies that can detect monocytes can be used as the antibodies of the present invention for detecting monocyte markers, and such antibodies bind to (1) HIDE1 protein, (2) a protein encoded by a nucleotide sequence that hybridizes under stringent conditions to the complementary sequence of HIDE 1 gene, or (3) a polypeptide fragment with at least eight consecutive amino acid residues of the protein of (1) or (2). Of these, antibodies that recognize antigenic regions of HIDE1 protein that are exposed on monocyte surfaces are particularly preferred as antibodies of the present invention for detecting monocyte markers.

Herein, "HIDE1" or "HIDE1 protein" refers to polypeptides encoded by a HIDE1 gene, including isolated natural proteins and recombinant proteins obtainable by expressing the gene using an appropriate expression system. The nucleotide sequence of mouse HIDE1 gene is shown in SEQ ID NO: 1, and the amino acid sequence of the protein encoded by the gene is shown in SEQ ID NO: 2. Mouse HIDE1 splice variants lacking a transmembrane domain were also found. An amino acid sequence of the splice variants is shown in SEQ ID NO: 4. The nucleotide sequence of an mRNA encoding the variants is shown in SEQ ID NO: 3. Both the transmembrane and secretory proteins described above are comprised in the definition of a HIDE1 protein described herein. The "HIDE1 protein" described herein encompasses not only the two types of mouse protein but also other variants, isoforms, and homologs of other mammals including humans.

The amino acid sequence of human HIDE1 is shown in SEQ ID NO: 6, and the nucleotide sequence encoding human HIDE1 is shown in SEQ ID NO: 5. Isoforms and variants of mouse and human HIDE1, and homologs of other mammals can be prepared, for example, by obtaining genes that encode an isoform, variant, or homolog from an appropriate cDNA library or genomic library using conventional gene cloning techniques such as hybridization and PCR, using as a probe or primer or the like a gene or a gene fragment encoding mouse or human HIDE1; and then expressing the obtained genes by known methods.

Nucleotide sequences that hybridize under stringent conditions to a complementary sequence of a HIDE1 gene have high homology to the HIDE1 gene, and are thus expected to encode proteins functionally equivalent to HIDE1. The antibodies of the present invention for detecting monocyte markers thus comprise antibodies that bind to proteins encoded by such nucleotide sequences. Furthermore, the antibodies of the present invention also comprise antibodies that recognize and specifically bind to an above-described HIDE1 protein or a fragment which is a portion of a protein encoded by a nucleotide sequence that hybridizes under stringent conditions to a complementary sequence of a HIDE 1 gene.

Specifically, stringent hybridization conditions of the present invention include, for example, conditions whereby hybridization is carried out using 5x SSC at 25°C in the absence of formamide. Preferably, hybridization is carried out at 25°C using 6x SSC in the presence of 40% formamide. More preferably, hybridization is carried out at 40°C using 5x SSC in the presence of 50% formamide. Post-hybridization washing is carried out, for example, at 37°C using 2x SSC. Preferably, washing is carried out at 55°C using 1x SSC. More preferably, washing is carried out at 60°C using 1x SSC.

When an antibody recognizes and binds to a protein, but binding to other proteins is not substantially detectable under the same conditions, that antibody can specifically recognize that protein. The degree of binding between an antibody and a protein can be assessed quantitatively based on immunoassay principles. Specifically, the specificity of an antibody can be assessed using methods such as FACS and ELISA. When FACS is used, the degree of antibody binding can be compared based on the number of positive cells. Alternatively, when ELISA is used, the degree of antibody binding can be compared based on the signal intensity produced by the labeled antibody. It is safe to conclude that an antibody specifically recognizes a target protein when the results of a quantitative evaluation of binding activity show that antibody cross-reactivity between the two proteins is 50% or less, for example, 20% or less, typically 10% or less, or 5% or less. For example, when an antibody binds to transformed cells expressing the human HIDE1 gene of SEQ ID NO: 5, but binding to the host cells used for the transformation is undetectable under the same conditions, the antibody can specifically recognize human HIDE1.

Particularly preferable fragments include, for example, fragments comprising a hydrophilic region or surface-constituting region of a HIDE1 protein; however, any fragment can be used, as long as it retains the antigenicity of a HIDE1 protein or a protein encoded by a nucleotide sequence that hybridizes under stringent conditions to the complementary sequence of a HIDE 1 gene. Thus, the antibodies of the present invention for detecting monocyte markers comprise antibodies that bind to a polypeptide fragment with eight or more amino acid residues (for example, 8, 9, 10, 11, 12, or 15 amino acid residues) which is a fragment of a HIDE1 protein or a protein encoded by a nucleotide sequence that hybridizes under stringent conditions to the complementary sequence of a HIDE1 gene.

Any antibody can be used as an antibody of the present invention, as long as it can detect an HIDE1 antibody. The antibodies include polyclonal antibodies, monoclonal antibodies, chimeric antibodies such as humanized antibodies, antibody fragments comprising a variable region (Fab, Fab', F(ab')2, Fv, and such), multispecific antibodies, and single-chain antibodies (scFv). The antibodies of the present invention may be modified by PEG or the like, as required. Further, as required, it is possible to label the antibodies with an appropriate enzyme (acetylcholine esterase, alkaline phosphatase, β-galactosidase, glucose-6-phosphate dehydrogenase, peroxidase, maltose-binding enzyme, glutathione transferase, and such), biotin, green fluorescence protein (GFP), radiolabel, fluorescent label, or the like, so that detection can be achieved without using a secondary antibody. When labeled with biotin, the antibodies can be recovered based on the binding between biotin and avidin, streptavidin, or the like. When the antibodies are fluorescently labeled, cells bound to the antibodies can be separated by a cell sorter using the fluorescence signal as an indicator. When bound to magnetic particles, the antibodies can be separated using a magnetic field or magnet. Further, if required, the antibodies of the present invention may be bound to an appropriate solid phase carrier, or the like.

The antibodies of the present invention can be produced by known methods. For example, when appropriate immune animals are immunized with HIDE1 protein or an antigenic fragment thereof, polyclonal antibodies recognizing HIDE1 can be obtained from the immune animals. The immune animals used for the purpose described above are not particularly limited, and any animal may be used as long as it can produce an antibody of the present invention. In general, animals belonging to Rodentia (mice, rats, hamsters, and such), Lagomorpha, Primates (cynomolgus monkeys, Rhesus monkeys, hamadryas baboons, chimpanzees, and such) are used to produce antibodies. Alternatively, for obtaining human antibodies, transgenic animals having the repertoire of human antibody genes may be used as immune animals.

Antigens used to prepare the antibodies of the present invention include the above-described (1) HIDE1 proteins, (2) proteins encoded by a nucleotide sequence that hybridizes under stringent conditions to a complementary sequence of a HIDE1 gene, and (3) polypeptide fragments with at least eight consecutive amino acid residues from a protein of the above (1) or (2). Particularly preferable antigen fragments are, for example, portions of HIDE1 proteins that are exposed on monocyte surfaces. For example, in the amino acid sequence of human HIDE1 shown in SEQ ID NO: 6, the extracellular domain corresponds to the amino acid sequence from position 27 to 117. Thus, polypeptides comprising the amino acid sequence from position 27 to 117 of human HIDE1 sequence, and polypeptides comprising an amino acid sequence of eight consecutive amino acids or more selected from the amino acid sequence from position 27 to 117 of human HIDE1 sequence are preferred antigen fragments.

Such antigens can be administered to immune animals, for example, by injection or such, in combination with an adjuvant if required, to immunize the animals. For immunization, antigens are preferably administered several times at fixed intervals. Sera are collected from the immunized animals and may be used as polyclonal antibodies or further purified if required.

It is also possible to prepare monoclonal antibodies by cloning antibody-producing cells from animals confirmed to produce an antibody with a desired activity. More specifically, spleens are excised from immunized animals; immune cells are isolated from the spleens; the cells are immortalized; and cells producing a desired monoclonal antibody are selected from the cells. A standard method for immortalizing antibody-producing immune cells comprises preparing hybridomas by fusing the cells with appropriate myeloma cells (see, for example, Methods Enzymol. (1981) 73: 3-46). Methods that immortalize cells by introducing an oncogene are also known. The obtained antibody-producing cells can be cultured and the culture supernatant yielded can be used as a monoclonal antibody or purified as required. Alternatively, the cells confirmed to produce a desired monoclonal antibody can be intraperitoneally transplanted to mice or such to collect desired monoclonal antibodies from the mice ascites by.

Alternatively, antibodies can be prepared by cloning antibody-encoding genes from the obtained antibody-producing cells using genetic engineering techniques. Once the antibody-encoding genes are cloned, chimeric antibodies, humanized antibodies, multispecific antibodies, scFv, and the like can be prepared. Such recombinant antibodies are also comprised in the antibodies of the present invention. Further, the antibodies of the present invention for detecting monocyte markers also comprise antibody fragments. Such antibody fragments can be produced by treating the polyclonal or monoclonal antibodies described above with an enzyme such as papain or pepsin. Alternatively, such antibody fragments can be obtained by preparing a polynucleotide chain that encodes an antibody fragment and inserting it into an expression vector, then expressing the fragment using conventional methods.

The antibodies of the present invention, comprising antibody fragments, can be collected and purified using protein A, protein G, or such. The antibodies can also be purified by using combinations of standard protein purification methods (ethanol precipitation, salting out, various types of chromatographic methods, gel electrophoresis, gel filtration, ultrafiltration, recrystallization, acid extraction, distillation, dialysis, immunoprecipitation, solvent extraction, solvent precipitation, ammonium sulfate precipitation, and such). The concentration of a yielded antibody can be determined by known methods such as absorbance measurements and enzyme-linked immunosorbent assays (ELISAs). Further, the antigen-binding activity of an antibody can be determined by absorbance measurements, fluorescence antibody methods, enzyme immuno assays (EIAs), radioimmunoassays (RIAs), ELISAs, or such. The activity of an antibody can also be evaluated using commercially available assay systems such as BIAcore (Pharmacia).

### [Detection and/or isolation of monocytes]

Since the antibodies of the present invention bind to monocytes, they can be used to detect monocytes in a sample or to obtain (isolate) monocytes. Specifically, the present invention provides methods for detecting and isolating monocytes. Monocytes can be detected by contacting an antibody of the present invention with a blood cell sample predicted to contain monocytes, and then detecting cells that bind to the antibody. Meanwhile, monocytes can be isolated by contacting an antibody of the present invention with a blood cell sample predicted to contain monocytes, and then collecting cells that bind to the antibody.

The methods of the present invention for detecting monocytes can be used in the diagnosis of diseases in which monocytes are involved. For example, since monocytes are known to increase in the circulatory system in inflammatory diseases, post-transplantation rejection, monocytic leukemia, infectious disease recovery phases, or such, such symptoms can be diagnosed by detecting monocytes in the peripheral blood or such. Further, since monocytes can differentiate into macrophages, dendritic cells, and the like, monocytes isolated by the methods of the present invention can be used in cell immunotherapy or such after being differentiated into macrophages and dendritic cells.

Herein, a "blood cell sample" can include, but is not limited to, for example, bone marrow, peripheral blood, and cord blood. A particularly preferred blood cell sample is peripheral blood. Such blood cell samples can be obtained from appropriate individuals. Herein, an individual refers to an individual whose tissue differentiation is completed and that can survive independently of its mother, and includes individuals at every growth stage, including not only adults but also individuals immediately after birth. It is not important whether the individuals from which the blood cell samples used in the present invention are collected are dead or alive, as long as proliferative cells can be separated from them. Thus, blood cell samples used in the present invention can be obtained from living individuals or individuals in a state of brain death or cardiac arrest.

Individuals for obtaining blood cell samples are vertebrates, for example, humans, mice, rats, rabbits, chickens and so on. Humans and mice are particularly preferred subjects. When aiming to use isolated monocytes in cell immunotherapy, for example, the blood cell sample used to isolate the monocytes is preferably collected from the actual patient to be treated. The risks of rejection reactions and infection by infectious pathogens can be reduced by using the patient's own cells.

The binding reaction between HIDE1 and the antibodies can be detected using immunoassay principles. In typical immunoassays, either protein or antibody is immobilized and detection is achieved after separating unreacted components. Methods for immobilizing cells or antibodies are known. For example, they can be immobilized directly onto solid phase by chemical linking or physical adsorption. Alternatively, when an antibody of the present invention is biotinylated, it is also possible to indirectly immobilize the antibody onto a solid phase which has adsorbed avidin, streptavidin, or the like. When an antibody is bound to magnetic particles, not only the antibody but also cells bound to the antibody can be quickly and conveniently detected and isolated using a magnet. Alternatively, when using an antibody that recognizes multiple antigens, such as a multispecific antibody, the antibody is bound to the HIDE1 on monocytes, and then other antigens can be bound to the antibody. Alternatively, antibodies can be immobilized onto a solid phase via protein A or G or the like.

The timing of antibody immobilization is not particularly limited and an antibody may be immobilized before, after, or simultaneously upon contact with a sample. An arbitrary solid phase may be used to immobilize the antibodies. Such solid phases include membranous, particulate, or fibrous carriers, which are made of glass; organic polymers such as polystyrene; and inorganic materials such as silica gel, alumina, and activated carbon. For example, the antibodies of the present invention may be immobilized onto the inner wall of a reaction container such as a plate, dish, and test tube, or to a bead.

Immunological methods for detecting or quantifying monocytes using the antibodies of the present invention for detecting monocyte markers include, for example, fluorescence antibody methods (see Monoclonal Antibodies: Principle and Practice, 3rd ed. (1996) Academic Press), ELISAs, RIAs, immunohistochemical staining such as immunocytological staining and immunohistological staining, (see, for example, the ABC method and CSA method; Monoclonal Antibodies: Principle and Practice, 3rd ed. (1996) Academic Press), Western blotting, and immunoprecipitation.

In ELISA, an antibody is labeled with an enzyme such as a peroxidase, whose substrate can be detected easily, or which catalyzes a reaction generating a detectable product, and then the concentration of the substrate or product or such is determined using an absorptiometer after reacting the enzyme with the substrate. Sandwich ELISA, which is one ELISA method, uses two types of antibodies that bind to different antigen epitopes, where one of the two is labeled with an enzyme. In RIAs, an antibody is radiolabeled so that it can be detected and quantified using a scintillation counter. In immunohistochemical staining, tissues, cells, or the like are reacted with an antibody labeled with a fluorescent substance or enzyme or such, and then pigment or such produced by the fluorescent or enzymatic reaction is observed using a microscope to investigate the tissue or cellular localization of a substance with which the antibody reacts. In immunoprecipitation, an antibody is reacted to cells, and then a carrier that specifically binds with immunoglobulin, such as protein G-Sepharose, is added to the reaction solution to precipitate the antigen-antibody complex.

A particularly preferred method for detecting and/or isolating monocytes comprises detecting and isolating cells of interest with a cell sorter using an antibody fluorescently labeled with fluorescein isothiocyanate (FITC), phycoerythrin, or such, using the fluorescence signal as an indicator. When antibodies that bind to different cell surface antigens are labeled with dyes with different fluorescent wavelengths and used in combination, cells can be selected using multiple cell surface antigens. In an alternative preferred method, cells of interest can be captured onto magnetic particles by reacting cells with antibody-immobilized magnetic particles. The cells bound to magnetic particles are separated using a magnetic device such as MACS (Daiichi Pure Chemicals Co.), and then the cells of interest can be collected. Such methods, comprising selecting cells using a single cell surface antigen and separating them using magnetic particles, are simple, and therefore particularly preferred as the methods of the present invention for detecting and/or isolating monocytes.

Isolated cells can be preferably cultured in any of various culture media (RPMI, IMDM, and such) known to those skilled in the art, supplemented with serum and amino acids. The culture is preferably conducted at 37°C under sterile conditions. Other culture condition s can be determined appropriately by those skilled in the art, depending on the purposes and such of the cells.

CD14 is known as a monocyte marker, and dendritic cells derived from CD14-positive monocytes are also being used in cell immunotherapy (Pickl et al., J. Immunol. (1996) 157:3850-9; Jefford et al., Blood (2003) 102: 1753). When monocytes are differentiated into dendritic cells, expression levels of both cell-surface CD14 and HIDE1 are reduced, although HIDE1 expression clearly fades more rapidly (two to three days after induction of differentiation) than CD14 expression. Specifically, there is a CD14-negative, HIDE1-positive stage in the process of differentiation from monocytes to dendritic cells. Thus it is suggested that the HIDE1 of the present invention may be expressed more specifically in monocytes than the conventional monocyte marker, CD14. Therefore, the methods of the present invention for detecting and/or isolating monocytes using HIDE1 as an indicator are expected to yield a purer monocyte population. In one embodiment, the present invention provides a monocyte population isolated using HIDE1 as a marker, where the monocyte population is purer than that yielded using a conventional marker.

### [Kits]

The antibodies of the present invention for detecting monocyte markers can form monocyte detection or isolation kits, in combination with substances required for detecting and/or isolating monocytes, and so on. For example, the antibodies of the present invention can form such kits, in combination with reagents, devices, and the like required to detect antibodies. For example, the kits may include: secondary antibodies for detecting the antibodies of the present invention; when the antibodies of the present invention are labeled with an enzyme,the kits may include the substrate in the reaction catalyzed by the enzyme; and when the enzyme has been bound to magnetic particles, the kits may include a magnet or such. Furthermore, in combination with the antibodies of the present invention, media suitable for monocyte culture, cytokines required for differentiation from monocytes to macrophages or dendritic cells, and others may constitute the kits. A manual is preferably attached to the kits, describing directions for use of the antibodies included in the kits for detecting and isolating monocytes, for the culture of the isolated monocytes, and for the induction of differentiation, and so on.

### [Induction of monocyte differentiation]

The action of appropriate cytokines is known to differentiate monocytes into macrophages (Nature (1987) 325:262-5; Nature (1986) 323: 86-9; J. Immunol. (1988) 140:1345-9; Jpn. J. Cancer Res. (1989) 80: 59-64), dendritic cells (see, for example, Thurnher et al., Exp. Hematology (1997) 25: 232-7; Schuler and Steinman, J. Exp. Med. (1997) 186: 1183-7), osteoclasts (Lacey et al., Endocrinol. (1988) 136: 2367-76; Mundy, Bone and Miner. Res. (1993) 8: S505-10; Japanese Patent Application Kokai Publication (JP-A (Kokai)) H11-196864 (unexamined, published Japanese patent application), or such. Differentiation of the monocytes isolated using the antibodies of the present invention for detecting monocyte markers can also be induced by the known techniques. Thus, in one embodiment, the present invention provides methods that comprise isolating monocytes using an antibody of the present invention, and then differentiating the isolated monocytes into macrophages, dendritic cells, osteoclasts, and such.

The present invention also revealed that HIDE1 expression was reduced or abolished in dendritic cells derived and differentiated from monocytes of human peripheral blood using GM-CSF and IL-4, and macrophage-like cells differentiated from THP-1, a monocytic cultured cell line, by phorbol ester, a differentiation-inducing factor. Thus, the differentiation of monocytes into macrophages or dendritic cells can be confirmed by monitoring the expression level of HIDE 1 during *in vitro* induction of macrophages or dendritic cells from monocytes. Therefore, the present invention provides methods for inducing macrophages or dendritic cells from monocytes, which comprise confirming cell differentiation using HIDE1 expression level.

The methods of the present invention for inducing dendritic cells from monocytes *in vitro* comprise the steps of: (1) culturing monocytes in the presence of a differentiation inducing factor for dendritic cells, and (2) detecting HIDE1 expression in the cultured cells using an antibody of the present invention. In addition, the methods of the present invention for inducing macrophages from monocytes *in vitro* comprise the steps of: (1) culturing monocytes in the presence of a differentiation inducing factor for macrophages, and (2) detecting HIDE 1 expression in the cultured cells using an antibody of the present invention. In both methods, a reduced HIDE1 expression level in step (2) indicates the monocytes are differentiated into dendritic cells or macrophages.

Dendritic cells (DCs) can be induced from monocytes by known methods (see, for example, J. LeukocyteBiol. (1996) 59: 208-18; Thurnher et al., Exp. Hematology (1997) 25: 232-7; Schuler and Steinman, J. Exp. Med. (1997) 186: 1183-7). Cytokines used to induce the differentiation of monocytes into DCs by the known methods can also be used as differentiation inducing factors for dendritic cells in the methods of the present invention for inducing DCs from monocytes. In particular, it has been reported that human monocytes cultured in the presence of granulocyte-macrophage colony stimulating factor (GM-CSF) and IL-4 have the ability to ingest antigens (phagocytosis) and transmit that antigen information to T cells, thereby activating the T cells (J. Leukocyte Biol. (1996) 59: 208-18). Thus, the combination of GM-CSF and IL-4 is particularly preferred as a differentiation inducing factor for a dendritic cell in the methods of the present invention.

DCs are classified into immature and mature DCs, according to their differentiation stages. Mature dendritic cells are characterized by the induction of T cell growth as well as the expression of CD80, CD86, CD83, MHC-I, MHC-II and so on. The phagocytic activity is stronger in immature DCs and weaker in mature DCs. The ability to present antigens to T cells accords with the expression levels of CD40, CD80, CD86, MHC-I, and MHC-II, which are involved in this ability. The ability is weak in immature DCs, but stronger in mature DCs. DCs confirmed to have differentiated from monocytes using an antibody of the present invention can also be further classified using these properties and/or markers by which immature and mature DCs are discriminated as indicators.

It has been reported that cytotoxic macrophages can be induced by culturing monocytes isolated from human peripheral blood in a culture medium containing serum in the presence of IL-1, IL-2, tumor necrosis factor (TNF), interferon γ(IFN- γ), or the like for 18 to 48 hours (Nature (1987) 325: 262-5; Nature (1986) 323: 86-9; J. Immunol. (1988) 140: 1245-9; Jpn. J. Cancer Res. (1989) 80: 59-64). JP-A (Kokai) H05-130863 reported that extremely cytotoxic macrophages could be yielded by using IL-2 and IL-1 or CSF-1 in combination. Cytokines used to induce the differentiation of monocytes into macrophages in these known methods can also be used as differentiation inducing factors for macrophages in the methods of the present invention for inducing macrophages from monocytes. Phorbol ester, used in Examples, is a particularly preferred differentiation inducing factor for macrophages in the methods of the present invention.

The monocytes used to induce dendritic cells and macrophages are not particularly limited, and include monocytic cultured cell lines THP-1, HL60, U937, and the like, as well as monocytes obtained from peripheral blood, cord blood, bone marrow, and such. The differentiation of monocytes is preferably induced in various culture media (RPMI, IMDM, and such) known to those skilled in the art, which are supplemented with serum and amino acids. The cells are preferably cultured under sterile conditions at 37°C. Induction of the differentiation of monocytes into dendritic cells is known to take five to seven days, and induction of the differentiation into macrophages is known to take 18 to 24 hours. Therefore, it is preferable to continue the culture for a period of five days or longer to achieve the differentiation into dendritic cells, or for 18 hours or longer to achieve the differentiation into macrophages. Other culture conditions can be determined appropriately by those skilled in the art depending on the purpose and such of the cells.

### [Methods for obtaining dendritic cells and macrophages]

Dendritic cells and macrophages can be obtained from blood cell samples by using the above-described methods for isolating monocytes and for inducing dendritic cells or macrophages from monocytes. Specifically, the present invention provides methods for obtaining dendritic cells and macrophages. It is known that dendritic cells can be used to prevent and/or treat tumors, autoimmune diseases such as rheumatism, or to relieve rejection reactions after organ transplantation, and dendritic cells obtained by the methods of the present invention can also be used for these purposes.

The use of macrophages has been proposed to treat spinal cord damage, or to treat and prevent tumors, infectious diseases, autoimmune diseases, and immunodeficiency diseases. Macrophages obtained by the methods of the present invention can also be used for these kinds of purposes. Further, it is known that macrophages have strong biosynthetic abilities and secrete biologically active, important macromolecules, including cytokines, growth factors, inflammatory mediators, proteases, and protease inhibitors. In addition, macrophages at a given stage of differentiation have only a limited lifetime and also have the property of migrating to inflammation sites and such, and thus it has also been proposed that such macrophages can be candidate cells for gene therapy (Japanese Patent Kohyo Publication No. (JP-A (Kohyo)) 2001-504683 (unexamined Japanese national phase publication corresponding to a non-Japanese international publication). Macrophages obtained by the methods of the present invention can also be used as such cells for gene therapy.

The methods of the present invention for obtaining dendritic cells (DCs) comprise the steps of: (1) culturing blood cells in the presence of a differentiation inducing factor for DCs; (2) contacting the cultured cells with an antibody for HIDE1 marker detection of the present invention, detecting HIDE1 expression, and judging that monocytes are differentiated into DCs when the HIDE1 expression level is reduced; and (3) isolating cells judged to be differentiated into DCs. The only requirement is that the blood cells to be differentiated into DCs comprise monocytes. However, to obtain more homogeneous DC populations, it is preferable to use isolated monocytes as a starting material.

Preferred blood cells comprising monocytes can be prepared, for example, by (1) contacting a blood cell sample with an antibody of the present invention for detecting a HIDE1 marker, and then (2) collecting blood cells bound to the antibody. Blood cell samples to be used for this purpose are not particularly limited, and any blood cell sample may be used as long as it comprises monocytes. Such blood cell samples include, for example, bone marrow, peripheral blood, and cord blood; and particularly preferably include peripheral blood. Such blood cell samples can be collected from appropriate individuals. However, when DCs prepared from isolated monocytes are administered to patients for treating or preventing disease, it is preferable to use a blood cell sample collected from the patient being administered, to avoid the risk of rejection and infection by infectious pathogens.

Thus, in one embodiment, the present invention provides methods for obtaining DCs, which comprise the steps of: (1) contacting a sample of collected blood cells with an antibody of the present invention; (2) collecting blood cells bound to the antibody; (3) culturing the collected blood cells in the presence of a differentiation inducing factor for DCs; (4) contacting the cultured cells with an antibody of the present invention, detecting HIDE1 expression, and judging that monocytes are differentiated into DCs when the expression level of HIDE1 is reduced; and (5) isolating the cells judged to be differentiated into DCs.

DCs are classified into immature and mature DCs according to their differentiation stages. Mature DCs are characterized by induction of T cell growth, and the expression of CD80, CD86, CD83, MHC-I, MHC-II and so on. The phagocytic activity is stronger in immature DCs but weaker in mature DCs. The ability to present antigens to T cells accords with the expression levels of CD40, CD80, CD86, MHC-I, and MHC-II, which are involved in this ability. The ability is weak in immature DCs, but stronger in mature DCs. DCs confirmed to have differentiated from monocytes using an antibody of the present invention can also be further classified using these properties and/or markers by which immature and mature DCs are discriminated as indicators.

In immune responses that occur upon invasion of a foreign antigen into the living body, DCs phagocytose the foreign antigens, migrate into lymph nodes, and present the antigens to lymphocytes. Thus, diseases involving the invasion of foreign antigens can be treated by injecting immature DCs directly into areas where the foreign antigens exist in the body. Therapeutic methods comprising injecting DCs directly into lesions such as tumors (dendritic cell injection (DCI)), are being performed, and DCs obtained by the methods of the present invention can also be used to treat tumors by direct injection into such lesions.

In addition, methods are known that use immature DCs to treat and prevent autoimmune diseases such as rheumatism, and to relieve rejection in organ transplantation, and such. For example, it is believed that the administration of immature DCs can result in IL-10 production or differentiation of IL-10-producing suppressor T cells from naïve T cells. IL-10 has the activity of inducing anergy (unresponsiveness) in T cells that attack autologous cells or organs derived from donors. More specifically, IL-10 has the activity of suppressing the development and activity of type 1 helper T cells and killer cells. It is understood that when immature DCs are administered, damage to autologous tissues and graft rejection is relieved based on this mechanism. DCs obtained by the methods of the present invention can also be used in the methods described above.

Further, methods using the DCs' antigen presenting ability are also known, whereby DCs ingest antigens *in vitro* prior to their administration to the body; and then these DCs are used in cell immunotherapy. Thus, in one embodiment of the present invention, the methods for obtaining DCs include methods further comprising the step of allowing cells, which have been judged to be differentiated into DCs, to ingest an antigen. DCs can ingest the antigen by contacting the antigen with the DCs, for example, by adding it to the DC culture medium. A cytokine such as TNF-α can also be added to the medium when contacting DCs with antigen molecules. To ensure antigen presentation by DC, antigens are contacted with DCs for several hours to several days, preferably for four to 48 hours. During this contact the medium may be changed or cytokines and antigens may be further added as required.

The antigens to be introduced into DCs include antigen components obtained from patients and known antigens produced artificially. For example, if aiming to suppress cancer recurrence and metastasis, an antigen extracted from an autologous cancer (a cancer cell lysate, acid-extracted peptide, or such) may be used as the antigen to be ingested by the DCs; or when there is a known tumor-specific antigen associated with a target cancer, an artificially produced tumor-specific antigen polypeptide may be used as the antigen to be ingested by the DCs. When aiming for cancer prevention, known tumor-specific antigens may be used. Alternatively, when aiming for cancer treatment, tumor-specific antigens or antigens extracted from an autologous cancer (cancer cell lysate, acid-extracted peptide, or such) can be used.

The present inventors found that, in addition to monocytes, bone marrow-derived dendritic cells could also be isolated by collecting HIDE1-positive cells from human peripheral blood samples. Specifically, not only monocytes but also dendritic cells can be collected when HIDE1 is used as a marker to obtain- these precursor monocytes for inducing dendritic cells. It is a novel finding obtained by the present inventors that peripheral blood contains HIDE1-positive bone marrow-derived dendritic cells, although in small numbers. Specifically, the present invention provides methods for isolating and/or detecting monocytes and bone marrow-derived dendritic cells in peripheral blood, which comprise the steps of:
(1) contacting a peripheral blood sample with an anti-HIDE1 antibody; and
(2) collecting the blood cells that bound to the antibody in step (1), which comprise monocytes and bone marrow-derived dendritic cells.

Alternatively, the present invention provides reagents comprising anti-HIDE1 antibodies, which are used to isolate and/or detect monocytes and bone marrow-derived dendritic cells in peripheral blood. The present invention also relates to uses of anti-HIDE1 antibodies to produce reagents for isolating and/or detecting monocytes and bone marrow-derived dendritic cells in peripheral blood.

The methods of the present invention for obtaining macrophages comprise the steps of:
(1) culturing blood cells in the presence of a differentiation inducing factor for macrophages; (2) contacting the cultured cells with an antibody of the present invention for detecting a HIDE1 marker, detecting HIDE1 expression, and judging that monocytes are differentiated into macrophage-like cells when the expression level of HIDE 1 is reduced; and (3) isolating the cells judged to have differentiated into macrophage-like cells. The only requirement is that the blood cells to be differentiated into macrophages comprise monocytes. However, to prepare more homogeneous macrophage populations, it is preferable to use isolated monocytes as a starting material.

Preferred blood cells comprising monocytes can be prepared, for example, by (1) contacting a blood cell sample with an antibody of the present invention for detecting a HIDE1 marker, and then (2) collecting blood cells bound to the antibody. Blood cell samples to be used for this purpose are not particularly limited, and any blood cell sample may be used as long as it comprises monocytes. The blood cell samples include, for example, bone marrow, peripheral blood, and cord blood; and particularly preferably include peripheral blood. Such blood cell samples can be collected from appropriate individuals.

However, when macrophages prepared from isolated monocytes are administered to patients for treating or preventing disease, it is preferable to use a blood cell sample collected from the patient being administered, to avoid the risk of rejection and infection by infectious pathogens. Thus, in one embodiment, the present invention provides methods for obtaining macrophages, which comprise the steps of: (1) contacting a sample of collected blood cells with an antibody of the present invention; (2) collecting blood cells bound to the antibody; (3) culturing the collected blood cells in the presence of a differentiation inducing factor for macrophages; (4) contacting the cultured cells with an antibody of the present invention, detecting HIDE1 expression, and judging that monocytes are differentiated into macrophage-like cells when the expression level of HIDE1 is reduced; and (5) isolating the cells judged to be differentiated into macrophages.

It is known that macrophages have strong biosynthetic ability, secrete biologically active, important macromolecules, including cytokines, growth factors, inflammatory mediators, proteases, protease inhibitors, active oxygen, and nitrogen monoxide, and have cytotoxicity against tumor cells, phagocytotic activity, antimicrobial activity, and such. Since macrophages have these kinds of characteristics, adoptive immunotherapy has been proposed, in which macrophages are activated *ex vivo* before being administered to patients. In one embodiment of the present invention, the methods for obtaining macrophages further comprise activating cells judged to be differentiated into macrophages. Macrophage-activation factors that are known to activate the functions of macrophages can be used to activate macrophages. Macrophages can be activated, for example, by adding a macrophage activation factor such as IFN-γ, IFN-α/β, IL-1, TNF, GM-CSF, or macrophage migration inhibitory factor (MIF), to the medium.

### [Collection and acquisition of lymphocytes]

The present invention revealed that HIDE1 is mainly expressed in monocytes but not in lymphocytes. Thus, lymphocytes can be collected from samples such as peripheral blood, which comprise lymphocytes and monocytes, by removing the monocytes from the sample by binding with the anti-HIDE1 antibodies of the present invention. Specifically, the present invention provides methods for collecting lymphocytes, which comprise the steps of: (1) contacting an antibody of the present invention with a blood cell sample predicted to contain lymphocytes, and (2) collecting blood cells that are not bound to the antibody.

Lymphocytes collected by the methods of the present invention can be used to prevent and treat tumors and infectious diseases, especially viral infections and the like, where lymphocytes are known to be effective. Known methods of adoptive immunotherapy comprise activating *ex vivo* autologous lymphocytes obtained from patients and then administering them to the patients. Thus, in one embodiment of the present invention, lymphocytes collected using an antibody of the present invention can be activated *ex vivo* to prepare activated lymphocytes.

The blood cell samples from which lymphocytes are collected are not particularly limited, and any blood cell sample can be used as long as it comprises monocytes. Such blood cell samples include, for example, bone marrow, peripheral blood, and cord blood; peripheral blood is particularly preferable. Such blood cell samples can be collected from appropriate individuals. However, when collected lymphocytes are administered to a patient to treat or prevent diseases, it is preferable to use a blood cell sample collected from the patient being administered, to avoid the risk of rejection reaction and infection by infectious pathogens. Various cytokines are known to have the activity of activating lymphocytes. For example, it is known that lymphokine-activated killer (LAK) cells with strong antitumor activity are yielded by reacting peripheral blood lymphocytes with IL-2 (activated lymphocyte therapy; J. Immunol. (1984) 132: 2123-8; J. Exp. Med. (1982) 155: 1823-41). Thus, LAK cells can be obtained by using IL-2 to activate lymphocytes collected by the methods of the present invention.

### [Administration of cells]

Monocytes, dendritic cells, macrophages, and lymphocytes obtained by the above-described methods of the present invention can be administered to patients to treat and prevent various diseases, to improve QOL, or for other purposes. Thus, the present invention provides methods for administering patients with one or more of these cells, therapeutic and preventive methods using these cells, and uses of these cells relating to treatment and prevention. Such cells isolated, obtained, or collected by any of the methods described above are cultured, amplified, and/or activated, if required. Cells of interest can be collected and, if required, washed, fractionated, concentrated, and so on, and then administered to patients. The cells are administered to appropriate sites, selected depending on each case and cell type, and are administered intravenously, intraperitoneally, intrathoracically, intratumorally, intraarterially, or by other route. Those skilled in the art can adjust the dose of each cell based on the patient's physical constitution, sex, age, symptoms and such, and the type of cell. The cells may be suspended in an appropriate vehicle when administered to patients. Preferred vehicles used to suspend the cells include physiological salines.

Diseases targeted by activated lymphocyte (LAK) therapies include cancers and malignant tumors such as sarcomas, and the therapies are performed to prevent recurrence (prevention of recurrence after extirpation of cancer lesions by surgical treatment), to treat diseases, and to improve QOL. Furthermore, the therapies have also been found to be effective against viral infections such as hepatitis C and B, and are also used against viral infections to potentiate patient immunity, prevent cancer development, and so on. Specifically, an appropriate amount of blood (about 15 ml) is collected from patients, lymphocytes are removed according to the methods of the present invention, the lymphocytes are amplified as much as possible by about two weeks of culture, and activated lymphocytes are returned to the patient's body.

When aiming to prevent recurrence of malignant tumors after extirpation of lesions, LAK cells are administered six times at about two-week intervals, and then monthly for two to five years. The therapies can also be applied to recurrent cancers and cancers that are not operable because of their locations. In such cases, LAK cells are administered approximately weekly, and if effects are observed, administration is preferably continued. Alternatively, LAK therapy can be used alongside treatments using anti-cancer agents and so on to produce synergistic effects and reduce adverse effects. When the therapies are used in combination with anti-cancer agents and so on, it is preferable to collect the blood required for the therapy prior to carrying out the other treatment. Lymphocytes collected or activated by the methods of the present invention may also be administered to patients using similar procedures to those conventionally used to administer lymphocytes.

Dendritic cell (DC) therapies are also used to prevent and treat recurrences of cancers and malignant tumors such as sarcomas. DCs are also important for conferring tolerance to rejection after organ transplantation and to autoimmune diseases. Methods used to achieve DC immunotherapy comprise administering cells obtained from a patient's blood or such directly into lesions of tumors or such, or allowing DCs to ingest tumor-specific antigens. The methods for allowing DCs to ingest tumor-specific antigens comprise providing components extracted from tumor cells obtained from patients, or providing known artificially-produced tumor-specific antigens.

In particular, these therapies are used to prevent recurrence after extirpation of cancers by surgery, or to treat metastatic cancers to the liver, lung, lymph nodes, and under the skin or such, as well as recurrent cancers. Basically, DCs which have ingested antigens are injected intradermally into areas surrounding the lymph nodes. The intrathoracic or intraperitoneal route may be selected depending on the type, location and such of the tumor. When the cells are administered directly into lesions of tumors or the like, CTs may be used as required. Some reports describe the use of dendritic cells to treat and prevent gastrointestinal cancers, malignant melanoma, hematopoietic tumors such as B cell tumor and chronic myelogenous leukemia, breast cancer, and the like. The cell dose used previously for direct intratumoral administration of DCs is about 1 x 10⁸ cells. DCs prepared by the methods of the present invention can also be administered to patients using similar procedures to those conventionally used for administering DCs.

Macrophages are being used to treat and/or prevent tumors, infectious diseases (particularly viral infections), autoimmune diseases, and immunodeficiency diseases. Furthermore, macrophage-based therapies for spinal cord damage are also known. Macrophages obtained by the methods of the present invention can also be administered to patients using similar procedures to those conventionally used for administering macrophages.

Hereinbelow, the present invention will be specifically described using Examples.

All the prior art documents cited herein are incorporated by reference.

### Examples

### 1. Preparation of anti-HIDE1 antibodies

Monoclonal antibodies against a human homolog of HIDE1 were prepared by the following procedure: First, PCR was used to amplify a human HIDE1 gene from a placental cDNA library. The yielded PCR product was cloned into pcDNA3.1 (Invitrogen), an expression vector for cultured animal cells (pcDNA-hHIDE1). In the constructed vector, the C terminus of human HIDE 1 (hHIDE1) is fused with a Myc tag. Cells of the human cultured cell line 293T were transiently transfected with pcDNA-hHIDE1 using Lipofectamine 2000 (Invitrogen), and the yielded cells expressing hHIDE1 were used as antigens to immunize Balb/C mice. Lymphocytes collected from the lymph nodes of the immunized mice were fused with Myeloma P3U1. After ten days, supernatant from each clone cell was collected as samples. Cell supernatants that reacted with the hHIDE1-experssing cell line were selected by flow cytometry (FCM). Thus, hybridomas producing anti-HIDE1 antibodies were obtained.

### 2. Flow cytometry (FCM)

hHIDE1-expressing 293T and wild type 293T were each suspended in PBS containing 2 mM EDTA and 0.5% BSA, and were then plated in 96-well Flexible Plates (FALCON) at a cell density of 1 x 10⁵ cells/well. After centrifugation at 700xg and 4°C for two minutes (TOMY Multipurpose Refrigerated Centrifuge LX120), the supernatant was discarded. 5 µl of Fc Receptor Block was added to each well. After the plates were allowed to stand for five minutes, 50 µl of the hybridoma (1H12) supernatant was added to each well. The plates were allowed to stand at room temperature for 30 minutes. Isotopic control IgG2a (Immunotech) diluted ten times with PBS containing 2 mM EDTA and 0.5% BSA was used as a negative control. After the antibody reaction, 100 µl of PBS containing 2 mM EDTA and 0.5% BSA was added to each well. The plates were centrifuged at 700xg and 4°C for two minutes, and the supernatant was discarded; another cycle of this treatment was performed for washing. After washing, 40 µl of Goat F(ab')2 Fragment Mouse IgG (H+ L)-PE (Immunotech) diluted 200 times with PBS containing 2 mM EDTA and 0.5% BSA was added to cells in each well, and the plates were allowed to stand at room temperature for 30 minutes. After secondary antibody reaction, the plates were washed three times. The cells were suspended in 1 ml of PBS containing 2 mM EDTA and 0.5% BSA and were analyzed using a flow cytometer (Cytomics FC500 Beckman counter). The results are shown in Fig. 4a.

### 3. Staining of PBMCs with an anti-HIDE1 antibody

The reactivity of an anti-HIDE1 antibody to peripheral blood mononuclear cell (PBMC) fraction was investigated to study human HIDE1 expression in blood cells. First, PBMCs were isolated from human peripheral blood using HISTOPAQUE-1077 (Sigma). The PBMCs were treated by the same FCM procedure as described above in Section 2. More specifically, using flow cytometry the PBMCs were divided by two-dimensional fractionation in terms of cell size and light scattering into three cell populations ― lymphocytes, monocytes, and granulocytes ― and then simple stained with an anti-HIDE1 antibody. As a result, it was found that HIDE1 was expressed mainly in monocytes, but not in lymphocytes. Some weak expression was also observed in granulocytes, but the staining was significantly weaker than in monocytes (Fig. 5). This result strongly suggests the possibility that HIDE1 can be used as a novel monocyte marker.

### 4. Double staining of PBMCs using an anti-HIDE1 antibody and another differentiation marker

PBMCs were suspended in PBS containing 2 mM EDTA and 0.5% BSA, and plated in each well of 96-well Flexible Plates (FALCON) at a cell density of 1 x 10⁵ cells/well. After centrifugation at 700xg and 4°C for two minutes (TOMY Multipurpose Refrigerated Centrifuge LX120), the supernatant was discarded. 5 µl of Fc Receptor Block was added to each well. After the plates were allowed to stand for five minutes, 50 µl of supernatant of hybridoma (1H12) was added to each well. The plates were allowed to stand at room temperature for 30 minutes. Isotypic control IgG2a (Immunotech) diluted ten times with PBS containing 2 mM EDTA and 0.5% BSA was used as a negative control.

After the antibody reaction, 100 µl of PBS containing 2 mM EDTA and 0.5% BSA was added to each well. The plates were centrifuged at 700xg and 4°C for two minutes and the supernatant was discarded; another cycle of this treatment was then performed for washing. After washing, 40 µl of Goat F(ab')2 Fragment Mouse IgG (H+L)-FITC (Immunotech) diluted 200 times with PBS containing 2 mM EDTA and 0.5% BSA was added to the cells in each well. The plates were allowed to stand at room temperature for 30 minutes. After the secondary antibody reaction, the cells were washed three times. After washing, 50 µl of PE-labeled antibody diluted ten times with PBS containing 2 mM EDTA and 0.5% BSA was added to the cells in each well. The plates were allowed to stand for 30 minutes at room temperature. The antibodies used were CD11b, CD14, CD33, and Isotypic control IgG1 (Immunotech). The cells were washed three times, and transferred into FCM tubes. Each volume was adjusted to 1 ml with PBS. The samples were assayed using a flow cytometer.

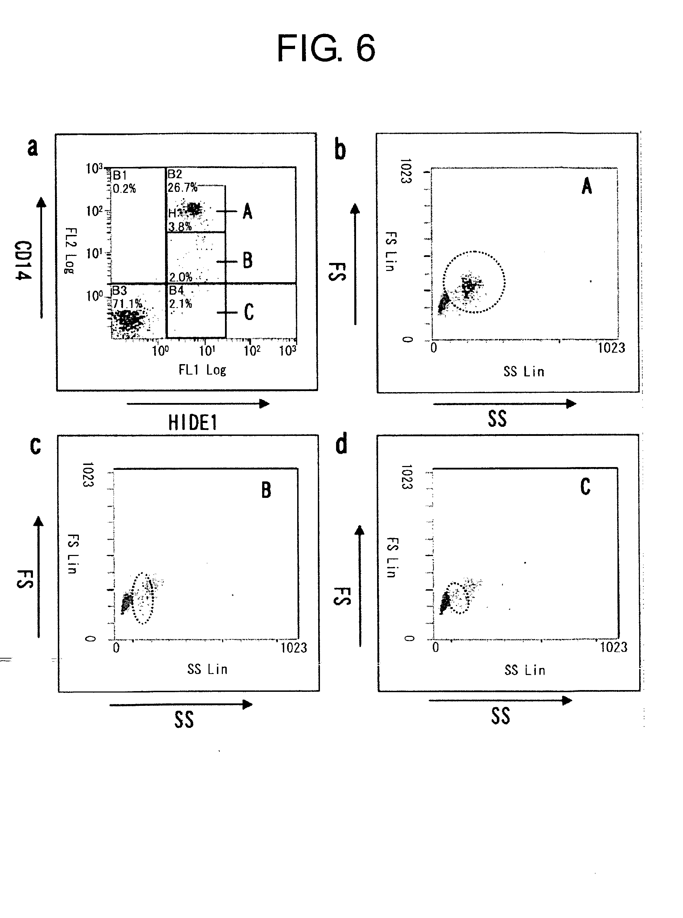

To more closely investigate HIDE1 expression in monocytes, double staining was carried out using CD14, a monocyte marker (ref.2), in combination with an anti-HIDE1 antibody (Fig. 6). HIDE1 was found to be expressed in 99% of CD14-positive cells (Figs. 6b and 6c). Since CD14-negative, HIDE1-positive cells account for 2% of PBMCs (Fig. 6d), the anti-HIDE1 antibody could possibly detect CD14-negative monocytes. Specifically, the anti-HIDE1 antibody is expected to be able to stain a broader range of monocytes than CD14.

Double staining with the anti-HIDE1 antibody and CD11b (ref.3), another monocyte marker, was conducted (Fig. 7), and the results showed that almost all cells in the strongly CD 11b-positive cell population were stained with HIDE 1 (Fig. 7b). The weakly CD 11b-positive cell population was divided into HIDE1-negative and HIDE1-positive populations. The weakly CD11b-positive, HIDE1-positive cell population mainly comprised monocytes (Fig. 7c), while the weakly CD11b-positive, HIDE1-negative cell population was a lymphocyte population (Fig. 7d). This result suggests that HIDE1 can stain monocytes more specifically than CD11b.

Double staining was carried out using the anti-HIDE1 antibody in combination with CD33 (ref.4), another monocyte marker (Fig. 8). It was found that CD33-positive cells account for 98% of HIDE1-positive cells and HIDE1-positive cells account for 94% of CD33-positive cells (Fig. 8b). These findings show that HIDE1 is an entirely new monocyte marker.

### 5. Staining of human cultured cells with an anti-HIDE1 antibody

Human cultured cells were stained with an anti-HIDE1 antibody using the same FCM procedure as described in Section 2. The human cultured cell lines used are THP-1, HL60, and U937. As a result HIDE1 was found to be expressed in human monocytic cultured cell lines such as HL60, U937, and THP-1 (Fig. 9). Thus, functional analyses of HIDE1 can be carried out using these human monocytic cultured cell lines.

The expression of mRNA was analyzed, and in mice HIDE1 was found to be expressed in dendritic cells derived from the spleen. Further, HIDE1 gene was also detectable in human spleen cDNA libraries, suggesting the possibility that HIDE1 is expressed in dendritic cells of human spleens (data not shown). However, HIDE1 was not expressed in dendritic cells derived and differentiated from human peripheral blood monocytes using GM-CSF and IL-4 (data not shown).

When THP-1, a monocytic cultured cell line, was differentiated into macrophage-like cell using phorbol ester, a differentiation-inducing factor, the expression level of HIDE1 was markedly reduced (Fig. 9d). These results strongly suggest that HIDE1 is expressed specifically in monocytes, and reinforce the fact that HIDE1 can be used as a monocyte marker. These results suggest the possibility that human HIDE1 is involved in the differentiation and/or growth of monocytes, and further that human HIDE1 may also be involved in myelocytic leukemia. The *in vivo* functions of HIDE1 can be clarified when ligands for HIDE1 are identified in the future.

### 6. Western blotting

Samples of hHIDE1-expressing 293T (H) and 293T (C) expressing another Myc tag fusion protein as a control were prepared in SDS-PAGE buffer, and electrophoresed using a 12.5% SDS-PAGE gel. Proteins fractionated by SDS-PAGE were transferred onto PVDF membrane (Millipore), and the blot membrane was treated with Block Ace (Dainippon Pharmaceutical Co. Ltd.) for two hours for blocking. Then, the blot membrane was incubated with hybridoma supernatant (3F12) at room temperature for one hour. To confirm HIDE1 expression in 293T, the blot membrane was incubated with anti-Myc-tag monoclonal antibody (MBL) 6000 times diluted with Block Ace.

The membrane was next washed with PBS containing 0.5% Tween20, and then incubated with anti-mouse IgG-POD conjugate (MBL) 3000 times diluted with Blue Buffer (20 mM HEPES, 1% BSA, 135 mM NaCl, 0.1% p-hydroxyphenyl acetic acid, 0.15% cathonCG, and 10 µg/ml bromophenol blue) at room temperature for one hour. After secondary antibody reaction, the membrane was washed and bands of interest were detected by chemiluminescence using SuperSignal (Pierce). The molecular weight of hHIDE1, estimated from the amino acid sequence, is 19 kDa. However, the extracellular domain of hHIDE1 is glycosylated at four sites, and as a result the detected-bands had shifted to positions at about 23 and 28 kDa (Fig. 4b).

### 7. Immunoprecipitation

0.5 mg of biotin (Pierce) was added to each of the cell suspensions (1 ml) of 293T (H) expressing hHIDE1, and 293T (C) expressing another protein (2 x 10⁷ cells /ml). The mixtures were stirred by inverting at 4°C for one hour. After centrifugation at 4° C and 200xg for five minutes the precipitates were washed four times with PBS. After washing, 1 ml of Lysis buffer (10 mM Tris-HCl (pH 7.5), 1% NP-40, 150 mM NaCl, x200 Protease inhibitor cocktail (Sigma)) was added to the precipitates. Each sample was mixed by inverting every five minutes for 30 minutes on ice. Then, the samples were centrifuged at 20,400xg for 15 minutes. The resulting supernatants were collected, and combined with 50 µl of rProteinA Sepharose FastFlow (Amersham Biosciences). The mixtures were stirred by inverting at 4°C for 30 minutes, and then centrifuged at 4°C and 600xg for one minute. The resulting supernatants were collected. The stirring was repeated once more.

Each supernatant was mixed with anti-HIDE1 antibody-immobilized Sepharose (prepared by combining 1 ml of supernatant of the antibody-producing hybridoma (3H3) with 50 µl of rProteinA Sepharose FastFlow, and rotating at 4°C for one hour). The resulting mixture was stirred by inverting at 4°C for one hour. After washing five times, the pellets were dissolved in SDS-PAGE buffer. The samples prepared were electrophoresed using a 12.5% SDS-PAGE gel. The proteins fractionated by the same procedure as in Section 6 were transferred onto a PVDF membrane. After the membrane was incubated with avidin-POD conjugate (MBL) diluted 10,000 times with blue buffer, bands of interest were detected by chemiluminescence using SuperSignal. The results are shown in Fig. 4c.

### 8. Double staining of PBMCs using an anti-hHIDE1 antibody and BDCA3, a marker for bone marrow-derived dendritic cells

PBMCs were collected from healthy donors and suspended in PBS containing 2 mM EDTA and 0.5% BSA. The cells were plated in 96-well Flexible Plates (FALCON) at a cell density of 1 x 10⁵ cells/well. After centrifugation at 700xg and 4°C for two minutes (TOMY Multipurpose Refrigerated Centrifuge LX120), the supernatant was discarded. 10 µl of Fc Receptor Block (1 mg/ml human IgG; Sigma) was added to each well. The plates were allowed to stand at room temperature for ten minutes. 50 µl of BDCA3-PE antibody (Miltenyi Biotec) diluted ten times with PBS containing 2 mM EDTA and 0.5% BSA was added to each well, and the plates were allowed to stand at 4°C for one hour. Isotypic control IgG1-PE (Immunotech) diluted ten times with PBS containing 2 mM EDTA and 0.5% BSA was used as a negative control. After the antibody reaction, 100 µl of PBS containing 2 mM EDTA and 0.5% BSA was added to each well. The plates were centrifuged at 700xg and 4°C for two minutes and the supernatants were discarded; another cycle of this treatment was performed for washing. After washing, 50 µl of purified anti-hHIDE1 antibody (1H12) labeled with FITC was added at a concentration of 2 µg/ml to the cells. The resulting mixtures were allowed to stand at 4°C for one hour. Isotypic control IgGI-FITC (Immunotech) diluted ten times with PBS containing 2 mM EDTA and 0.5% BSA was used as a negative control. After washing three times, each sample was transferred into a FCM tube, and the volume was adjusted to 1 ml with PBS. The samples were assayed using a flow cytometer. The results are shown in Fig. 10.

As a result, it was found that most BDCA3-positive cells were HIDE1-positive (enclosed by the broken line in Fig. 10b). These results show that HIDE1 is a marker for bone marrow-derived dendritic cells as well as a monocyte marker, suggesting the possibility that monocytes and bone marrow-derived dendritic cells can be collected simultaneously from peripheral blood by using antibodies against HIDE1.

### 9. Preparation of anti-mouse HIDE1 antibody

Mouse HIDE1 gene was amplified by PCR from a cDNA library of cultured cell DC2.4. The PCR product was cloned into pcDNA3.1 (Invitrogen), an expression vector for cultured animal cells (pcDNA-mHIDE1). The vector was designed so that the C terminus of mouse HIDE 1 (mHIDE1) was fused with Myc tag. pcDNA-mHIDE1 was transiently introduced into L cell, a mouse cultured cell line, using Lipofectamine 2000 (Invitrogen). Wister rats were immunized using the obtained mHIDE1-expressing cells as an antigen. Lymphocytes collected from lymph nodes of the immunized rats were fused with Myeloma P3U1. After ten days, the supernatant of each clone was sampled. Cell supernatants that reacted with the mHIDE1-experssing cell line were selected by flow cytometry and hybridomas producing anti-mHIDE1 antibodies were thus obtained. The flow cytometry is described in detail below.

### 10. Flow cytometry (FCM)

mHIDE1-expressing 293T and wild type 293T, or mHIDE1-expressing L-cells and wild type L-cells were each suspended in PBS containing 2 mM EDTA and 0.5% BSA, and plated in 96-well Flexible Plats (FALCON) at a cell density of 1 x 10⁵ cells/well. After centrifugation at 700xg and 4°C for two minutes (TOMY Multipurpose Refrigerated Centrifuge LX120), the supernatants were discarded. 50 µl of the hybridoma supernatant was added to each well. The plates were allowed to stand at room temperature for 30 minutes. Isotypic controls, IgGG1, 2a, and 2b (MBL), 100 times diluted with PBS containing 2 mM EDTA and 0.5% BSA, were used as negative controls.

After the antibody reaction, 100 µl of PBS containing 2 mM EDTA and 0.5% BSA was added to each well. The plates were centrifuged at 700xg and 4°C for two minutes, and the supernatant was discarded; another cycle of this treatment was performed for washing. After washing, 40 µl of Goat F(ab')2 Fragment Rat IgG (H+L)-PE (Immunotech) diluted 200 times with PBS containing 2 mM EDTA and 0.5% BSA was added to each well, and the plates were allowed to stand at room temperature for 30 minutes. After the secondary antibody reaction, the plates were washed three times. The cells were suspended in 1 ml of PBS containing 2 mM EDTA and 0.5% BSA, and analyzed using a flow cytometer (Cytomics FC500 Beckman counter).

### 11. Double staining of PBMCs using an anti-mHIDE1 antibody and a differentiation marker

To test the reactivity of an anti-mHIDE1 antibody to peripheral blood mononuclear cell (PBMC) fraction, PBMCs were isolated from the peripheral blood of c57BL6 mice using Lmpholyte-Mammal (CEDARLANE). PBMCs suspended in PBS containing 2 mM EDTA and 0.5% BSA were plated in 96-well Flexible Plats (FALCON) at a cell density of 1 x 10⁵ cells/well. After centrifugation at 700xg and 4°C for two minutes (TOMY Multipurpose Refrigerated Centrifuge LX120), the supernatants were discarded. 50 µl of 100 times diluted Fc Receptor Block (CD16/32 antibody; Pharmingen) was added to each well. The plates were allowed to stand at room temperature for 10 minutes. 50 µl aliquots of PE-labeled antibody diluted 100 times with PBS containing 2 mM EDTA and 0.5% BSA were added, and the plates were allowed to stand at 4°C for one hour. The antibodies used were CD11b, CD11c, and Isotypic controls IgG2a and 2b (Pharmingen).

After the antibody reaction, 100 µl of PBS containing 2 mM EDTA and 0.5% BSA was added to each well. The plates were centrifuged at 700xg and 4°C for two minutes, and the supernatants were discarded; another cycle of this treatment was performed for washing. After washing, 50 µl of a purified anti-mHIDE1 antibody (5F8) labeled with FITC was added to the cells at a concentration of 2 µg/ml, and the resulting mixtures were allowed to stand at 4°C for one hour. Isotypic control IgG2b-FITC (Pharmingen), diluted 100 times with PBS containing 2 mM EDTA and 0.5% BSA, was used as a negative control. The cells were washed three times, and transferred into FCM tubes. The volume was adjusted to 1 ml with PBS. The samples were assayed using a flow cytometer. The results are shown in Figs. 11a and 11b, and Figs. 12a and 12b.

### 12. Staining of mouse spleen cells with an anti-mHIDE1 antibody

To test the reactivity of an anti-mHIDE1 antibody to spleen cells, spleen cells were isolated from the spleens of c57BL6 mice. PBMCs were treated by the same procedure as in Section 10. The results are shown in Figs. 11c and 11d, and Figs. 12c and 12d.

Mouse peripheral mononuclear blood (PBMC) and mouse spleen cells were double-stained with an anti-mHIDE1 antibody (5F8) and a dendritic cell marker (CD11c). The results showed that a part of the CD11c-positive cells were HIDE1-positive (enclosed by the broken line in Figs. 12b and 12d). CD11c is also expressed in NK cells. It was confirmed that HIDE1 was not expressed in NK cells (data not shown).

The results described above show that like human HIDE1, mouse HIDE1 is expressed in peripheral blood monocytes (Fig. 11) and dendritic cells (Fig. 12) as well as in splenic monocytes and dendritic cells (Figs. 12 and 13). Since human HIDE1 distribution was confirmed to correlate with mouse HIDE1 distribution, it is thought that the *in vivo* functions of HIDE1 can be analyzed using mice in the future.

### Industrial Applicability

The present invention provided novel monocyte markers. Monocytes are cells which migrate in blood and which have phagocytotic activity, belonging to the group of mononuclear phagocytes. Once differentiated, monocytes remain in the bone marrow for only a short time before entering the circulation system, where they stay for several days. Monocytes then infiltrate tissues and body cavities, and differentiate into macrophages and dendritic cells. Monocytes are known to increase in the circulatory system during inflammatory responses, and thus monocyte detection is thought to be useful to detect inflammatory responses. Meanwhile, an increase in monocytes after organ transplantation suggests the possibility of tissue or graft rejection, and thus the monocyte detection of the present invention is useful for diagnoses after organ transplantation.

Since HIDE1 is expressed specifically in peripheral blood monocytes, HIDE1-positive monocytes can be collected from peripheral blood using a cell sorter, magnet, or such. Monocytes are precursor cells of dendritic cells and macrophages, and dendritic cells and macrophages can be prepared from monocytes *in vitro.* Dendritic cells derived from CD14-positive monocytes are used in cell immunotherapy (Pickl et al., J. Immunol. (1996) 157: 2850-9; Jefford et al., Blood (2003) 102: 1753). Monocytes selected using HIDE1 as a marker are also expected to be applicable to cell immunotherapy. Since HIDE1 is not expressed in lymphocytes it can be used as a tool to enrich lymphocytes by removing HIDE1-positive cells from peripheral blood using an anti-HIDE1 antibody.

Methods for treating tumors and viral infections using activated lymphocytes have been proposed. Thus, cells that can be used to treat and prevent cancers, viral infections, spinal cord damage, and various other diseases for which the administration of monocytes, macrophages, dendritic cells, or lymphocytes is effective, can be prepared efficiently by using an antibody that recognizes an HIDE1 marker of the present invention. Human HIDE1 may be involved in the differentiation and/or growth of monocytes, and may also be involved in myelocytic leukemias. When ligands for HIDE1 are identified, the *in vivo* functions of HIDE1 are expected to be further clarified.

## Claims

1. An antibody for detecting a monocyte marker, which binds to a protein or a polypeptide selected from the group of:
(1) an HIDE1 protein;
(2) a protein encoded by a nucleotide sequence that hybridizes to a complementary sequence of an HIDE1 gene under stringent conditions; and
(3) a polypeptide fragment with at least eight amino acid residues, wherein the fragment is derived from the protein of the above (1) or (2).

2. A method for detecting a monocyte, which comprises the steps of:
(1) contacting the antibody of claim 1 with a blood cell sample predicted to comprise a monocyte, and
(2) detecting a blood cell that has bound to the antibody in step (1).

3. A method for isolating a monocyte, which comprises the steps of:
(1) contacting the antibody of claim 1 with a blood cell sample predicted to comprise a monocyte, and
(2) collecting a blood cell that has bound to the antibody in step (1).

4. The method of claim 2 or 3, wherein the blood cell sample is peripheral blood, cord blood, or bone marrow.

5. A kit for detecting and/or isolating a monocyte, which comprises the antibody of
claim 1.

6. A method for inducing a dendritic cell from a monocyte *in vitro,* which comprises the steps of:
(1) culturing a monocyte in the presence of a differentiation inducing factor for a dendritic cell ; and
(2) contacting a cell cultured in step (1) with the antibody of claim 1, detecting HIDE1 expression, and judging that the differentiation of a monocyte into a dendritic cell is induced when the expression level of HIDE1 is reduced.

7. The method of claim 6, wherein the differentiation inducing factor for a dendritic cell is a combination of GM-CSF and IL-4.

8. A method for inducing a macrophage from a monocyte *in vitro,* which comprises the steps of:
(1) culturing a monocyte in the presence of a differentiation inducing factor for a macrophage; and
(2) contacting a cell cultured in step (1) with the antibody of claim 1, detecting HIDE1 expression, and judging that the differentiation of a monocyte into a macrophage-like cell is induced when the expression level of HIDE1 is reduced.

9. The method of claim 8, wherein the differentiation inducing factor for a macrophage is phorbol ester.

10. A method for obtaining a dendritic cell, which comprises the steps of:
(1) contacting a sample of collected blood cells with the antibody of claim 1;
(2) collecting a blood cell that has bound to the antibody in step (1);
(3) culturing the blood cell collected in step (2) in the presence of a differentiation inducing factor for a dendritic cell;
(4) contacting the cell cultured in step (3) with the antibody of claim 1, detecting HIDE1 expression, and judging that a monocyte is differentiated into a dendritic cell when the expression level of HIDE1 is reduced; and
(5) isolating as a dendritic cell the cell judged to be differentiated in step (4).

11. The method of claim 10, which further comprises the step of allowing the isolated dendritic cell to ingest an antigen.

12. The method of claim 10, wherein the isolated dendritic cell is used to prevent and/or treat a tumor.

13. The method of claim 12, which further comprises the step of allowing the isolated dendritic cell to ingest a tumor-specific antigen.

14. The method of claim 10, wherein the isolated dendritic cell is used to prevent and/or treat an autoimmune disease, or to relieve rejection after an organ transplantation.

15. A method for obtaining a macrophage, which comprises the steps of:
(1) contacting a sample of collected blood cells with the antibody of claim 1;
(2) collecting a blood cell that has bound to the antibody in step(1);
(3) culturing the blood cell collected in step (2) in the presence of a differentiation inducing factor for a macrophage;
(4) contacting the cell cultured in step (3) with the antibody of claim 1, detecting HIDE1 expression, and judging that a monocyte is differentiated into a macrophage-like cell when the expression level of HIDE1 is reduced; and
(5) isolating as a macrophage the cell judged to be differentiated in step (4).

16. The method of claim 15, which further comprises the step of activating the isolated cell.

17. The method of claim 15 or 16, wherein the isolated macrophage is used to treat a spinal cord damage, and/or to treat and/or prevent a tumor, infectious disease, autoimmune disease, or immunodeficiency disease.

18. A method for collecting a lymphocyte, which comprises the steps of:
(1) contacting the antibody of claim 1 with a blood cell sample predicted to comprise a lymphocyte; and
(2) collecting a blood cell that did not bind to the antibody in step (1).

19. A method for obtaining an activated lymphocyte, which comprises the steps of:
(1) contacting the antibody of claim 1 with a blood cell sample predicted to comprise a lymphocyte;
(2) collecting as a lymphocyte a blood cell that is not bound to the antibody;
(3) culturing the lymphocyte collected in step (2); and
(4) activating the lymphocyte cultured in step (3) and collecting the activated lymphocyte.

20. The method of claim 18 or 19, wherein the blood cell sample is peripheral blood, cord blood, or bone marrow.

21. The method of claim 19, wherein the activated lymphocyte is used to prevent and/or treat a tumor or infectious disease.
